# EUROPEAN PATENT APPLICATION

(11) **EP 1 739 419 A1**
(43) Date of publication of application: **03.01.2007**
(21) Application number: 05728584.3
(22) Date of filing: 01.04.2005
(51) Int. Cl.: G01N 27/64, G01N 27/62

(54) **METHOD OF MEASURING SUBSTANCE BY MASS SPECTROMETRY AND KIT THEREFOR**

(30) Priority: 13.04.2004 JP 2004118343; 24.05.2004 JP 2004153484; 07.09.2004 JP 2004259855; 20.12.2004 JP 2004368507; 20.12.2004 JP 2004368508; 20.12.2004 JP 2004368510
(71) Applicant: SHIMADZU CORPORATION, Kyoto-shi, Kyoto 604-8511 (JP)
(72) Inventor: MATSUO, Eiichi c/o Shimadzu Corporation, Kyoto-shi, Kyoto 604-8511 (JP); WATANABE, Makoto c/o Shimadzu Corporation, Kyoto-shi, Kyoto 604-8511 (JP); OJIMA, Noriyuki c/o Shimadzu Corporation, Kyoto-shi, Kyoto 604-8511 (JP); TODA, Chikako c/o Shimadzu Corporation, Kyoto-shi, Kyoto 604-8511 (JP); KUYAMA, Hiroki c/o Shimadzu Corporation, Kyoto-shi, Kyoto 604-8511 (JP); NISHIMURA, Osamu c/o Shimadzu Corporation, Kyoto-shi, Kyoto 604-8511 (JP)
(74) Representative: Pohlmann, Eckart
(86) International application number: PCT/JP2005/006890
(87) International publication number: WO 2005/100975

(57) **Abstract**

The present invention provides method and kit for measuring substance using mass spectrometry. In more detail, the present invention provides method for measuring hydrophobic peptides using MALDI mass spectrometer, method for selective measurement of specific substances from a mixture by mass spectrometry, and method and kit for quantitative analysis of protein. A method for measuring a peptide with a MALDI mass spectrometer using α-cyano-3-hydroxycinnamic acid or 3-hydroxy-4-nitrobenzoic acid as a matrix. A mass spectrometric method comprising: in a mass spectrometry specifically ionizing a specific substance in a mixture containing the specific substance and the other substance by using a matrix that is more likely to ionize the specific substance than the other substance, to selectively measure the specific substance from the mixture. A method and a kit for quantitative analysis of protein.

## Description

### TECHNICAL FIELD

The present invention relates to method and kit for measuring substance using mass spectrometry, and includes first, second and third inventions.

The first invention is method for measuring hydrophobic peptides using MALDI (Matrix Assisted Laser Desorption/Ionization) mass spectrometer and relates to a field of proteome analysis using a mass spectrometer.

The second invention is method for selective measurement of specific substances from a mixture by MALDI mass spectrometry and relates to mass spectrometry and quantitative proteome analysis by a MALDI method.

The third invention is method and kit for quantitative analysis of protein and relates to a proteome analysis (global analysis of protein) using a stable isotope.

### BACKGROUND ART

The background art of the first invention will be described as follows.

### <Quantitative analysis>

In the field of proteome analysis (global analysis of protein), a PMF (Peptide Mass Finger Printing) analysis method in which a two-dimensional gel electrophoresis and a mass spectrometer are combined has been commonly used. As a next-generation proteome analysis method which will be an alternative to the PMF, approaches using stable isotopes have been proposed. For example, Salvatore Sechi and Yoshiya Oda, Quantitative proteomics using mass spectrometry, Current Opinion in Chemical Biology, 2003, 7, 70-77 discloses a quantitative proteomics using the mass spectrometry using an ICAT (Isotope-Coded Affinity Tag) reagent. Disclosed in Hiroki Kuyama, Makoto Watanabe, Chikako Toda, Eiji Ando, Koichi Tanaka and Osamu Nishimura, An Approach to Quantitative Proteome Analysis by Labeling Tryptophan Residues, Rapid Communications in Mass Spectrometry, 2003, 17, 1642-1650 and international publication WO 2004/002950 pamphlet is a method developed by the present inventors (this method is hereinafter referred to as "NBS method"), including: modifying a tryptophan residue in a protein or a peptide with stable isotope-labeled and non-labeled 2-nitrobenzenesulfenyl chloride (NBSCl) serving as a labeling reagent; enzymatically digesting the resultant protein or peptide with trypsin to obtain a peptide mixture; enriching peptides having nitrobenzenesulfenyl (NBS)-modified tryptophan from the peptide mixture, using hydrophobic column chromatography technique; and conducting a quantitative analysis.

### <Sequencing by MS/MS analysis>

In the PMF method, mass values of a plurality of peptide fragments obtained by enzymatic digestion of one protein are detected by mass spectrometer, and the mass values are compared with a set of mass values of theoretical segments on the database, whereby the protein is identified. In contrast, in the NBS method, an original protein is identified by subjecting labeled and enriched peptides to a MS/MS analysis to determine their sequences and searching the database for proteins containing such sequences. Accordingly, in the NBS method, the sequencing by the MS/MS analysis is essential process for identifying a protein in addition to the quantitative analysis by commonly-used mass spectrometry.

The MS/MS analysis is performed by the three steps: 1) selection of a peptide to be sequenced, referred to as "precursor ion"; 2) fragmentation of the selected precursor ion; and 3) detection of mass values of resultant fragment ions. Examples of the mass spectrometer capable of conducting such a MS/MS analysis include: a tandem mass spectrometer having two mass separation units connected with each other, dedicated to selection of precursor ion and to detection of fragment ion; mass spectrometer utilizing decay by the own internal energy of ion (PSD: post-source decay); ion trapping mass spectrometer capable of conducting MS analysis more than once (MSⁿ analysis) by trapping ions; and FTICR mass spectrometer generating mass spectrometric signals through FT (Fourier transform) of data obtained by using the ion cyclotron resonance (ICR) technique.

In a matrix assisted laser desorption/ionization (MALDI) mass spectrometers, molecules (generally organic compounds) called matrix mixed with an analyte are heated by absorption of energy of a laser beam, vaporized and ionized. Instant vaporization of the matrix surrounding analyte molecules brings as a result the analyte molecules as well released into a gas phase almost concurrently. At this time, electrons or protons are transferred between the analyte molecule and the matrix, and thus the ionization of analyte molecule is achieved. Concurrently, the energy is partially transferred to the analyte molecule as internal energy.

As described above, the matrix also serves as a dispersing agent for the analyte molecule, while assisting vaporization and ionization of the analyte molecule. Therefore, affinity between matrix and analyte molecule would be important for ionization of the sample molecule with high efficiency. For this reason, an optimum organic compound for each analyte has been searched and used. For example, in measurement of a peptide, α-cyano-4-hydroxycinnamic acid (4-CHCA) is widely and generally used.

In the MALDI-IT (Matrix Assisted Laser Desorption/Ionization - Ion Trap) mass spectrometer, MALDI-IT-TOF (Matrix Assisted Laser Desorption/Ionization - Ion Trap - Time of Flight) mass spectrometer (for example, AXIMA-QIT (manufactured by SHIMADZU Corporation), and MALDI-FTICR (Matrix Assisted Laser Desorption/Ionization - Fourier Transform Ion Cyclotron Resonance) mass spectrometer, the analyte molecule that have been ionized by the MALDI method is enclosed once in a cell, and then detection and selection of ions or a MS/MS analysis is performed. Therefore, the time required for a generated ion to reach the detector and undergo measurement is more than two or three orders longer than that required in a usual MALDI-TOF (Matrix Assisted Laser Desorption/Ionization - Time of Flight) mass spectrometer. The ionized analyte molecules have excess internal energy, so that they self-disintegrate gradually. In a practical PSD measurement with a MALDI-TOF mass spectrometer, a pseudo MS/MS analysis is conducted based on this principle.

Therefore, in the MALDI-IT, MALDI-IT-TOF, and MALDI-FTICR mass spectrometers, considerable internal energy received during ionization will lead an unfavorable result that during measurement a number of undesired fragment ions are detected. From this point of view, using 4-CHCA as a matrix is not favorable in the MALDI-IT, MALDI-IT-TOF, and MALDI-FTICR mass spectrometers because excess internal energy will be applied on the analyte during ionization. Therefore, the matrix that is practically selected as a default in an AXIMA-QIT apparatus is 2,5-dihydroxy benzoic acid (DHB) which is believed to give relatively small energy on the analyte molecules during ionization.

The background art of the second invention will be described as follows.

### <Mass spectrometry based on the MALDI method>

In a MALDI mass spectrometer, molecules (generally organic compounds) called "matrix" with an analyte are heated by absorption of energy of a laser beam, and vaporized and ionized. Due to instant vaporization of the matrix surrounding analyte molecules, the analyte molecules are also, as a result, almost simultaneously released into a gas phase. (At this time, electrons or protons are passed between the analyte molecules and the matrix, and thus the ionization of the analyte molecules is achieved.) In mass spectrometry according to the MALDI method, an optimum organic compound for each analyte to be measured is searched and used. For example, in measurement of a peptide, α-cyano-4-hydroxycinnamic acid (4-CHCA), 2,5-dihydroxybenzoic acid (DHB) are generally used.

### <Proteome analysis>

In the field of proteome analysis (global analysis of protein), a PMF (Peptide Mass Finger Printing) analysis method in which a two-dimensional electrophoresis and a mass spectrometer are combined is commonly used. As a next-generation proteome analysis method which will be an alternative to the PMF, for example, approaches using stable isotopes as disclosed in: Steven P. Gygi, Beate Rist, Scott A. Gerber, Frantisek Turecek, Michael H. Gelb and Ruedi Aebersold, Quantitative analysis of complex protein mixtures using isotope-coded affinity tags, Nature Biotechnology, 994-999, 17, 1999; Kirk C. Hansen, Gerold Schmitt-Ulms, Robert J. Chalkley, Jan Hirsch, Michael A. Baldwin and A. L. Burlingame, Mass Spectrometric Analysis of Protein Mixtures at Low Levels Using Cleavable 13C-Isotope-coded Affinity Tag and Multidimensional Chromatography, Molecular & Cellular PROTEOMICS, 299-314, 2, 2003; and, Salvatore Sechi and Yoshiya Oda, Quantitative proteomics using mass spectrometry, Current Opinion in Chemical Biology, 70-77, 7, 2003 have been contrived.

In Hiroki Kuyama, Makoto Watanabe, Chikako Toda, Eiji Ando, Koichi Tanaka and Osamu Nishimura, An Approach to Quantitative Proteome Analysis by Labeling Tryptophan Residues, Rapid Communications in Mass Spectrometry, 1642-1650, 17, 2003, and the international publication WO 2004/002950 pamphlet, a method developed by the present inventors (NBS method) is disclosed. The NBS method uses stable isotope-labeled 2-nitrobenzenesulfenyl chloride (NBSCl) (2-nitro [¹³C₆] benzenesulfenyl chloride) and non-labeled NBSCl (2-nitro [¹²C₆] benzenesulfenyl chloride). Specifically, the method includes the steps of: (1) preparing two states of protein samples, a protein sample I to be analyzed and its reference protein sample II; (2) modifying the protein sample I with either one of 2-nitro [¹³C₆] benzenesulfenyl chloride and 2-nitro [¹²C₆] benzenesulfenyl chloride, while modifying the protein sample II with the other one of 2-nitro [¹³C₆] benzenesulfenyl chloride and 2-nitro [¹²C_{6]} benzenesulfenyl chloride; (3) mixing the modified protein sample I and the modified protein sample II with each other; (4) removing unreacted reagents by using a desalting column; (5) subjecting the resultant mixture of modified proteins to reduction and alkylation followed by digestion into a peptide mixture containing modified peptide fragments and unmodified peptide fragments; (6) enriching/ separating the modified peptide fragments from the peptide mixture by using a hydrophobic chromatography column; and (7) conducting mass spectrometry.

### <Nitrotyrosine>

It is known that, in a living organism, nitrogen monoxide (NO) and highly-reactive nitrogen oxides generating therefrom (such as peroxynitrite) will react with proteins or nucleic acids to generate nitro compounds. For example, in the case of a protein, reaction occurs at a phenyl group which is a side chain of tyrosine to generate 3-nitrotyrosine. Tyrosine residues are frequently phosphorylated in a living organism. Phosphorylation has a role of a switch in critical events in a living organism such as signal transduction and cell death. For this reason, living organism-related substances having a nitrotyrosine receive attention not only as an index for generation of a reactive nitrogen oxide in a living organism (so-called "bio-marker") but also from the view point of biological activity.

A nitrating method of a tyrosine residue in a protein is disclosed in: A reagent for the nitration of tyrosine and tyrosyl residues of proteins, Journal of the American Chemical Society, 1966, 88, 4104-410; and, A reagent for the nitration of tyrosyl residues in proteins, Biochemistry, 1966, 5, 3582-3589.

The background art of the third invention will be described as follows.

In the field of proteome analysis (global analysis of protein), a PMF (Peptide Mass Finger Printing) analysis method in which a two-dimensional gel electrophoresis and a mass spectrometer are combined is commonly used. As a next-generation proteome analysis method which will be an alternative to the PMF, for example, approaches using stable isotopes as disclosed in: Steven P. Gygi, Beate Rist, Scott A. Gerber, Frantisek Turecek, Michael H. Gelb and Ruedi Aebersold, Quantitative analysis of complex protein mixtures using isotope-coded affinity tags, Nature Biotechnology, 994-999, 17, 1999; Kirk C. Hansen, Gerold Schmitt-Ulms, Robert J. Chalkley, Jan Hirsch, Michael A. Baldwin and A. L. Burlingame, Mass Spectrometric Analysis of Protein Mixtures at Low Levels Using Cleavable 13C-Isotope-coded Affinity Tag and Multidimensional Chromatography, Molecular & Cellular PROTEOMICS, 299-314, 2, 2003; and, Salvatore Sechi and Yoshiya Oda, Quantitative proteomics using mass spectrometry, Current Opinion in Chemical Biology, 70-77, 7, 2003 have been contrived.

In Hiroki Kuyama, Makoto Watanabe, Chikako Toda, Eiji Ando, Koichi Tanaka and Osamu Nishimura, An Approach to Quantitative Proteome Analysis by Labeling Tryptophan Residues, Rapid Communications in Mass Spectrometry, 1642-1650, 17, 2003, and the international publication WO 2004/002950 pamphlet, a method developed by the present inventors (NBS method) is disclosed. The NBS method uses stable isotope-labeled 2-nitrobenzenesulfenyl chloride (NBSCl) (2-nitro [¹³C₆] benzenesulfenyl chloride) and unlabeled NBSCl (2-nitro [¹² C₆] benzenesulfenyl chloride). Specifically, the method includes the steps of: (1) preparing two states of protein samples, a protein sample I to be analyzed and its reference protein sample II; (2) modifying the protein sample I with either one of 2-nitro [¹³ C₆] benzenesulfenyl chloride and 2-nitro [¹² C₆] benzenesulfenyl chloride, while modifying the protein sample II with the other one of 2-nitro [¹³C₆] benzenesulfenyl chloride and 2-nitro [¹²C₆] benzenesulfenyl chloride; (3) mixing the modified protein sample I and the modified protein sample II with each other; (4) subjecting the resultant mixture of modified proteins to reduction and alkylation followed by digestion into a peptide mixture containing modified peptide fragments and unmodified peptide fragments; (5) enriching/ separating the modified peptide fragments from the peptide mixture by using a hydrophobic chromatography column; and (6) conducting mass spectrometry.

One exemplary protocol of NBS method will be described below.
· Solubilize two series of protein samples, a test sample and a control sample, respectively, in a solution containing 0.1 w/v% SDS, and denature by heating (100°C, 3 min.).
· Add an acetic acid solution dissolving NBS (Heavy) reagent to one of the samples, and an acetic acid solution dissolving NBS (Light) reagent to the other of the samples, to cause NBS modification reaction respectively (room temperature, overnight).
· Mix both of the samples and remove unreacted reagents using a desalting column (LH-20).
· Dry the sample after desalting and resuspend in a solution containing 0.01 w/v% SDS.
· Add TCEP (Tris(2-carboxyethyl)phosphine hydrochloride) to cause reductive reaction (37°C, 30 min.).
· Add an iodoacetamide solution to cause alkylation reaction (room temperature, 45 min.).
· Add trypsin to cause site-specific cleavage (37°C, 16 hours).
· Enrich NBS-modified peptides using an enrichment column (LH-20).
· Analyze enriched fractions using a mass spectrometer.

### DISCLOSURE OF THE INVENTION

The object and the summary of the first invention will be described as follows.

### Object of the Invention

As described above, in the MALDI-IT, MALDI-IT-TOF, and MALDI-FTICR mass spectrometers, DHB is predominantly used as a matrix. However, in the case of measuring a sample containing hydrophobic peptides, the hydrophobic peptides cannot be effectively ionized even using the DHB.

It is an object of the present invention to provide a method capable of efficiently ionizing hydrophobic peptides in MALDI mass spectrometers, especially in MALDI-IT, MALDI-IT-TOF, and MALDI-FTICR mass spectrometers.

### Summary of the Invention

As a result of diligent research, the inventors have found that the above object of the present invention is achieved by using, as a matrix, α-cyano-3-hydroxycinnamic acid or 3-hydroxy-4-nitrobenzoic acid, and accomplished the present invention.

The present invention encompasses the following aspects.
(1) A method of measuring a peptide with a mass spectrometer having a MALDI (Matrix Assisted Laser Desorption/Ionization) ion source, using α-cyano-3-hydroxycinnamic acid or 3-hydroxy-4-nitrobenzoic acid as a matrix.
(2) The method of measuring a peptide according to the above (1), wherein when 3-hydroxy-4-nitrobenzoic acid is used as the matrix, the measuring is performed by using a mixed matrix of 3-hydroxy-4-nitrobenzoic acid and α-cyano-4-hydroxycinnamic acid.
(3) The method of measuring a peptide according to the above (1) or (2), wherein the peptide is a peptide that is chemically modified with a hydrophobic compound.
(4) The method of measuring a peptide according to any one of the above (1) to (3), wherein the peptide is a peptide that has an amino acid residue modified with a sulfenyl compound.
(5) The method of measuring a peptide according to any one of the above (1) to (4), wherein the peptide is a peptide that is derivatized with 2-nitrobenzenesulfenyl chloride.
   The term "peptide" used herein should be understood to include oligopeptides, polypeptides, and proteins.
(6) The method of measuring a peptide according to any one of the above (1) to (5), performed by a MALDI-IT (Matrix Assisted Laser Desorption/Ionization - Ion Trap) mass spectrometer.
(7) The method of measuring a peptide according to any one of the above (1) to (5), performed by a MALDI-IT-TOF (Matrix Assisted Laser Desorption/Ionization - Ion Trap - Time of Flight) mass spectrometer.
(8) The method of measuring a peptide according to any one of the above (1) to (5), performed by a MALDI-FTICR (Matrix Assisted Laser Desorption/Ionization - Fourier Transform Ion Cyclotron Resonance) mass spectrometer.
(9) The method of measuring a peptide according to any one of the above (1) to (8), wherein the matrix is used as a solution having a concentration of 1 mg/ml to a saturated concentration.
(10) The method of measuring a peptide according to any one of the above (2) to (9), wherein the α-cyano-4-hydroxycinnamic acid is used as a solution having a concentration of 1 mg/ml to a saturated concentration.
(11) The method of measuring a peptide according to the above (10), wherein the solution of 3-hydroxy-4-nitrobenzoic acid and the solution of α-cyano-4-hydroxycinnamic acid are used in combination in a volume ratio of 1:10 to 10:1.
   According to the present invention, it is possible to provide a method capable of efficiently ionizing hydrophobic peptides in MALDI mass spectrometers, especially in MALDI-IT, MALDI-IT-TOF, and MALDI-FTICR mass spectrometers.
   The object and the summary of the second invention will be described as follows.

### Object of the Invention

In the MALDI method, vaporization and ionization of a analyte/matrix mixture is conducted by irradiation with a laser beam, and the number of molecules that can be ionized in a single shot is limited. Accordingly, when the sample to be measured includes a plurality of molecule species, a molecular species existing at high abundance is ionized at higher probability, while a molecular species existing at low abundance is detected at less probability even if an amount exceeding a detection limit of the analyte exists. Also in the case where there is a difference in "ionization tendency" between analyte molecules to be measured, molecules having poor tendency to ionization can be detected with poor sensitivity. Therefore, it is important to enrich an objective molecule in advance in order to detect a molecular species that is hard to be ionized and a molecular species that exists in a small amount.

In the NBS method, one of other analyses that require quantification, detection of an NBS-modified molecule which is an objective molecule can be done without enrichment, however, the quantitativeness may be impaired if the NBS-modified molecule overlaps with irrelative molecules. Therefore, still in this case, it would be preferable to remove in advance the molecular species that are not modified with NBS by enrichment from the view point of improvement in quantitativeness.

It is an object of the present invention to provide a cost-effective and sensitive detection method capable of predominantly detecting an objective substance in mass spectrometry.

### Summary of the Invention

The present invention encompasses the following aspects.
(12) A mass spectrometric method comprising:
   in a mass spectrometry specifically ionizing a specific substance to be measured contained in a mixture sample containing the specific substance and a substance other than the specific substance by using a matrix that is more likely to ionize the specific substance than the substance other than the specific substance, to selectively measure the specific substance from the mixture.

   That is, the matrix of the present invention can specifically ionize the specific substance because it mainly ionizes the specific substance and hard to ionize the substance other than the specific substance.
(13) The mass spectrometric method according to the above (12), wherein the specific substance is a substance related to a living organism.
(14) The mass spectrometric method according to the above (13), wherein the substance related to a living body is selected from protein, peptide, sugar, and lipid.
(15) The mass spectrometric method according to any one of the above (12) to (14), wherein the specific substance is labeled with an isotope.
(16) The mass spectrometric method according to any one of the above (12) to (15), wherein the matrix can interact with the specific substance via van der Waals interaction.
(17) The mass spectrometric method according to any one of the above (12) to (16), wherein the specific substance is a π electron containing substance, and the matrix is a π electron containing substance.
   That is, in this aspect of the present invention, π-π electrons interaction between the specific substance to be measured and the matrix is used.
(18) The mass spectrometric method according to any one of the above (12) to (16), wherein the specific substance is a hydrophilic substance, and the matrix is a hydrophilic substance.
   That is, in this aspect of the present invention, hydrophilic interaction between the specific substance to be measured and the matrix is used.
(19) The mass spectrometric method according to any one of the above (12) to (17), wherein the specific substance is a hydrophobic substance, and the matrix is a hydrophobic substance.
   That is, in this aspect of the present invention, hydrophobic interaction between the specific substance to be measured and the matrix is used.
   The followings are inventions related to the above aspect (19).
(20) The mass spectrometric method according to the above (19), wherein the specific substance is a hydrophobic peptide or a hydrophobic protein.
(21) The mass spectrometric method according to the above (20), wherein the hydrophobic peptide or the hydrophobic protein has a benzene ring and/or an aromatic ring other than a benzene ring.
(22) The mass spectrometric method according to the above (20) or (21), wherein the hydrophobic peptide or the hydrophobic protein further has a nitro group.
(23) The mass spectrometric method according to any one of the above (20) to (22), wherein the hydrophobic peptide or the hydrophobic protein has a nitrobenzenesulfenyl group or a nitrophenyl group.
(24) The mass spectrometric method according to any one of the above (20) to (23), wherein the hydrophobic peptide or the hydrophobic protein is obtained by chemically modifying a peptide or a protein corresponding to the hydrophobic peptide or the hydrophobic protein by using a hydrophobic compound having a benzene ring, an aromatic ring other than a benzene ring, and/or a nitro group.
   This aspect encompasses the case where the peptide or the protein corresponding to the hydrophobic peptide or the hydrophobic protein itself is hydrophobic. In other words, this aspect encompasses the case where a hydrophobic peptide or a hydrophobic protein is derived into a peptide or protein having higher hydrophobicity through chemical modification using a hydrophobic compound.
(25) The mass spectrometric method according to the above (24), wherein the hydrophobic compound is a sulfenyl compound.
(26) The mass spectrometric method according to the above (25), wherein the sulfenyl compound is 2-nitrobenzenesulfenyl chloride.
(27) The mass spectrometric method according to any one of the above (19) to (26), wherein the matrix is a substituted compound of a benzene ring or an aromatic ring other than a benzene ring, having a functional group for transferring electric charges from/to the specific substance to be measured and a functional group for affording hydrophobicity to the matrix molecule itself.
(28) The mass spectrometric method according to the above (27), wherein the functional group for transferring electric charges is selected from carboxyl group, hydroxyl group, amino group, sulfate group, nitrate group, and aldehyde group.
(29) The mass spectrometric method according to the above (27) or (28), wherein the functional group for affording hydrophobicity is nitro group.
(30) The mass spectrometric method according to any one of the above (19) to (29), wherein the matrix is a nitrobenzoic acid derivative or a nitrophenol derivative.
(31) The mass spectrometric method according to any one of the above (19) to (30), wherein the matrix is a hydroxynitrobenzoic acid derivative.
(32) The mass spectrometric method according to any one of the above (19) to (31), wherein the matrix is selected from positional isomers of hydroxynitrobenzoic acid.
(33) The mass spectrometric method according to any one of the above (19) to (29), wherein the matrix is selected from 4-nitroaniline, 2,4-dinitroaniline, 2-bromo-4,6-dinitroaniline, 4-nitrophenol, 2-nitrophenol, 2,5-dinitrophenol, 4-nitrobenzoic acid, 3-hydroxy-4-nitrobenzoic acid, and 3-hydroxy-2-nitrobenzoic acid.
(34) The mass spectrometric method according to any one of the above (19) to (33), wherein as the matrix, a mixed matrix combined with α-cyano-4-hydroxycinnamic acid is used.
(35) The mass spectrometric method according to any one of the above (19) to (34), wherein the matrix is used as a solution of 1 mg/ml to a saturated concentration.
(36) The mass spectrometric method according to the above (34) or (35), wherein the α-cyano-4-hydroxycinnamic acid is used as a solution of 1 mg/ml to a saturated concentration.
(37) The mass spectrometric method according to the above (36), wherein the matrix solution and the solution of α-cyano-4-hydroxycinnamic acid are used in a volume ratio of 1:10 to 10:1.

According to the present invention, it is possible to provide a method capable of predominantly detecting an objective substance in mass spectrometry without enriching the objective substance to be measured.

The object and the summary of the third invention will be described as follows.

### Object of the Invention

A conventional NBS method developed by the present inventors is especially effectively applied when purified samples such as protein model are used. In order to provide a method for global quantitative analysis of protein that is effectively applied not only for purified samples as described above but also for unpurified samples such as biological samples, and achieves better detection sensitivity and quantitativeness than the conventional NBS method, the inventors of the present application accomplished the present invention.

### Summary of the Invention

The present invention encompasses the following aspects.
(38) A method for global quantitative analysis of protein comprising the steps of:
   (i) preparing two states of protein samples, a Protein sample I for analysis and a control Protein sample II;
   (ii) solubilizing the Protein sample I in a solution containing urea as a denaturing agent or in a solution containing guanidine hydrochloride as a denaturing agent, to obtain a solubilized Protein sample I, and
      separately solubilizing the Protein sample II in a solution containing urea as a denaturing agent or in a solution containing guanidine hydrochloride as a denaturing agent, to obtain a solubilized Protein sample II;
   (iii) subjecting the solubilized Protein sample I to modification reaction using either one of 2-nitro[¹³C₆]benzenesulfenyl chloride and 2-nitro[¹²C₆]benzenesulfenyl chloride, to obtain a modified Protein sample I, and
      separately subjecting the solubilized Protein sample II to modification reaction using the other one of 2-nitro[¹³C₆]benzenesulfenyl chloride and 2-nitro[¹²C₆]benzenesulfenyl chloride, to obtain a modified Protein sample II;
   (iv) mixing and desalting the modified Protein sample I and the modified Protein sample II, to obtain a desalted protein sample mixture;
   (v) resolubilizing the desalted protein sample mixture by using urea or guanidine hydrochloride, to obtain a resolubilized protein sample mixture;
   (vi) reducing and alkylating the resolubilized protein sample mixture, to obtain a reduced and alkylated protein sample mixture;
   (vii) subjecting the reduced and alkylated protein sample mixture to trypsin digestion in the presence of urea or guanidine hydrochloride, to obtain a peptide mixture containing modified peptide fragments and unmodified peptide fragments;
   (viii) separating the peptide mixture using a media having a phenyl group, to obtain enriched modified peptide fragments; and
   (ix) subjecting the enriched modified peptide fragments to mass spectrometry.
(39) The method according to the above (38), wherein in the step (ix), the mass spectrometry is conducted using a-cyano-3-hydroxycinnamic acid or 3-hydroxy-4-nitrobenzoic acid as a matrix.
(40) The method according to the above (38), wherein in the step (ix), when 3-hydroxy-4-nitrobenzoic acid is used as the matrix, the mass spectrometry is conducted using a mixed matrix of 3-hydroxy-4-nitrobenzoic acid and α-cyano-4-hydroxycinnamic acid.
(41) The method according to the above (39) or (40), wherein the matrix is used as a solution having a concentration of 1 mg/ml to a saturated concentration.
(42) The method according to the above (40) or (41), wherein α-cyano-4-hydroxycinnamic acid is used as a solution having a concentration of 1 mg/ml to a saturated concentration.
(43) The method according to the above (42), wherein the solution of 3-hydroxy-4-nitrobenzoic acid and the solution of α-cyano-4-hydroxycinnamic acid are combined in a volume ratio of 1:10 to 10:1 to be used.
(44) A kit containing 2-nitro[¹³C₆]benzenesulfenyl chloride, 2-nitro[¹²C₆]benzenesulfenyl chloride, and a media having a phenyl group.
(45) A kit for carrying out the method according to any one of the above (38) to (43), containing 2-nitro [¹³C₆]benzenesulfenyl chloride, 2-nitro[¹²C₆]benzenesulfenyl chloride, and a media having a phenyl group.
(46) The kit according to the above (44) or (45), further containing a denaturing agent.
(47) The kit according to any one of the above (44) to (46), further containing α-cyano-3-hydroxycinnamic acid, 3-hydroxy-4-nitrobenzoic acid, or α-cyano-4-hydroxycinnamic acid as a matrix, or a mixture of 3-hydroxy-4-nitrobenzoic acid and α-cyano-4-hydroxycinnamic acid as a mixed matrix.
(48) The kit according to the above (46) or (47), wherein the denaturing agent is urea or guanidine hydrochloride.
(49) The kit according to any one of the above (44) to (48), further containing at least one selected from the group consisting of a desalting column, filling gel for a desalting column, a reduction reagent, an alkylation reagent, trypsin, and a column for filling the media.

According to the present invention, a method capable of detecting an objective peptide from a biological sample with better sensitivity and quantitativeness in mass spectrometry can be provided.

### BRIEF DESCRIPTION OF THE DRAWINGS

Fig. 1 to Fig. 3 relate to the first invention.
Fig. 1 shows a MS spectrum (a) for comparison obtained by a conventional method, MS spectra (b) and (c) obtained by the method of the present invention in Example A-1;
Fig. 2 shows a product-ion mass spectrum obtained in Example A-1; and
Fig. 3 shows a MS spectrum (a-1) obtained by measuring with an AXIMA-QIT having an ion trap, and a MS spectrum (a-2) obtained by measuring with an AXIMA-CFR plus without an ion trap in Example A-2(a); and a MS spectrum (b-1) obtained by using a mixture of 3H4NBA and 4-CHCA as a matrix and a MS spectrum (b-2) obtained by using 3H4NBA as a matrix in Example A-2(b).
Fig. 4 to Fig. 13 relate to the second invention.

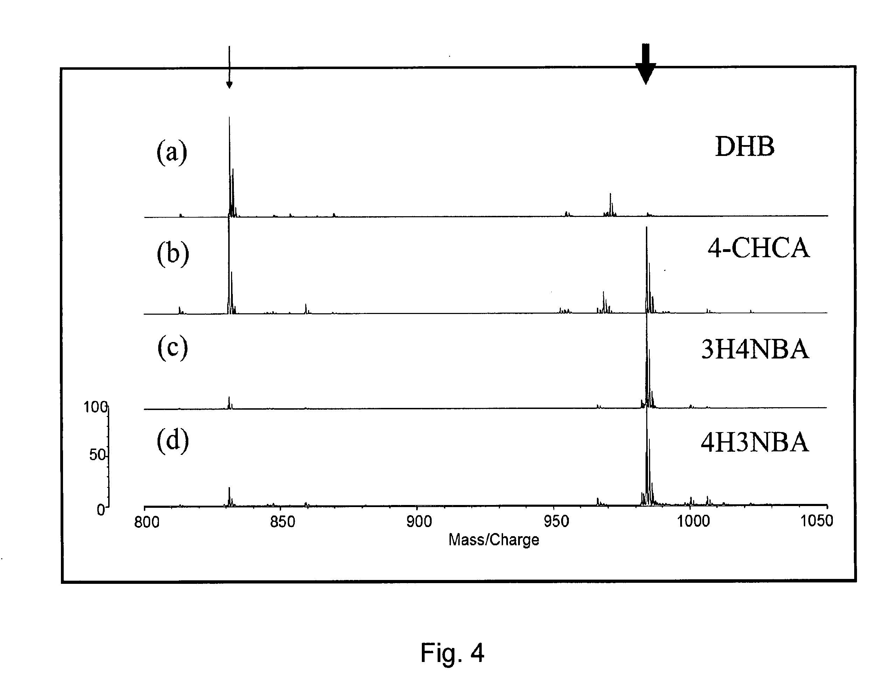

   Fig. 4 shows mass spectra ((a) and (b)) obtained by the conventional method and mass spectra ((c) and (d)) obtained by the present invention when the sample to be measured is a mixture of ACTH peptide and ACTH peptide modified with NBS Reagent (light);

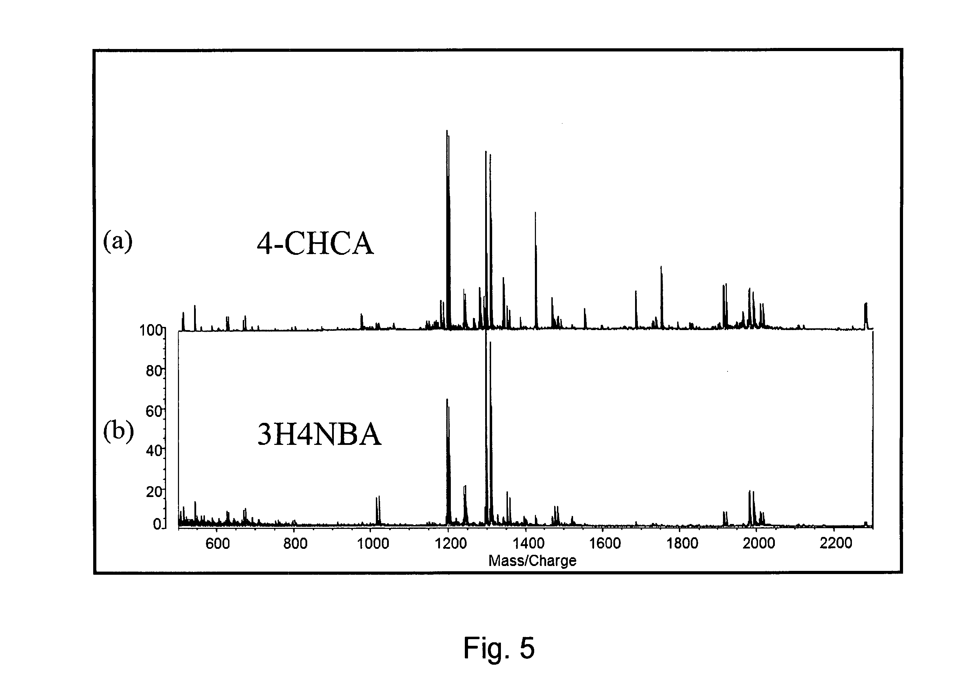

Fig. 5 shows a mass spectrum (a) obtained by the conventional method and a mass spectrum (b) obtained by the present invention when the sample to be measured is prepared by digesting a 1:1 mixture of NBS Reagent (heavy) modified- and NBS Reagent (light) modified- four purified proteins (ovalbumin, glyceraldehyde-3-phosphate dehydrogenase, lysozyme, and α-lactalbumin);

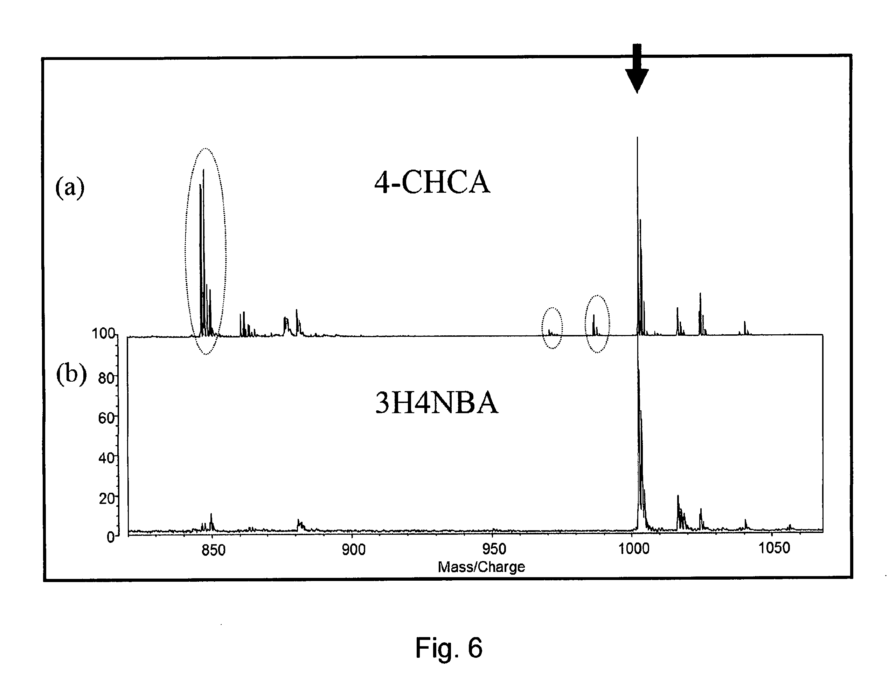

Fig. 6 shows a mass spectrum (a) obtained by the conventional method and a mass spectrum (b) obtained by the present invention when the sample to be measured is a mixture of DSIP peptides and DSIP peptides modified with NBS Reagent (light);

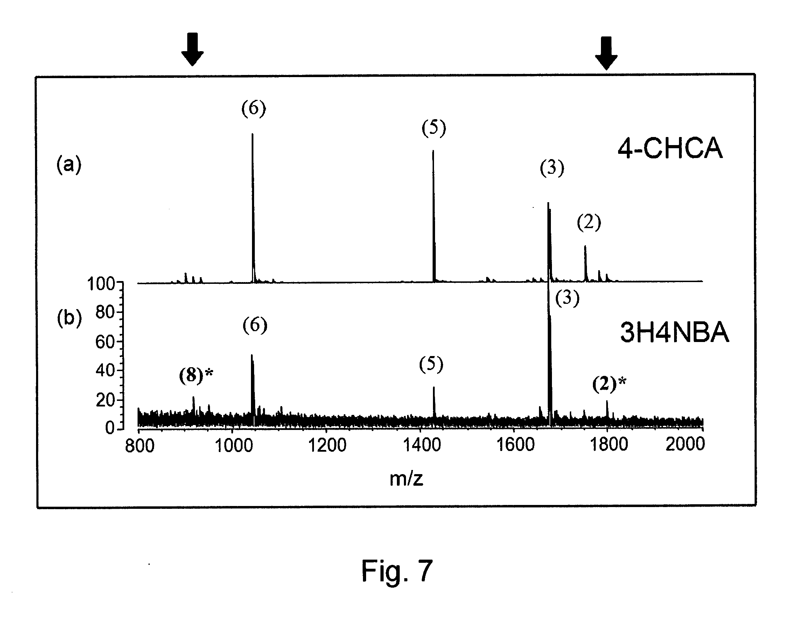

Fig. 7 shows a mass spectrum (a) obtained by the conventional method and a mass spectrum (b) obtained by the present invention when the sample to be measured is a mixture obtained by nitrating lysozyme followed by digesting with trypsin;

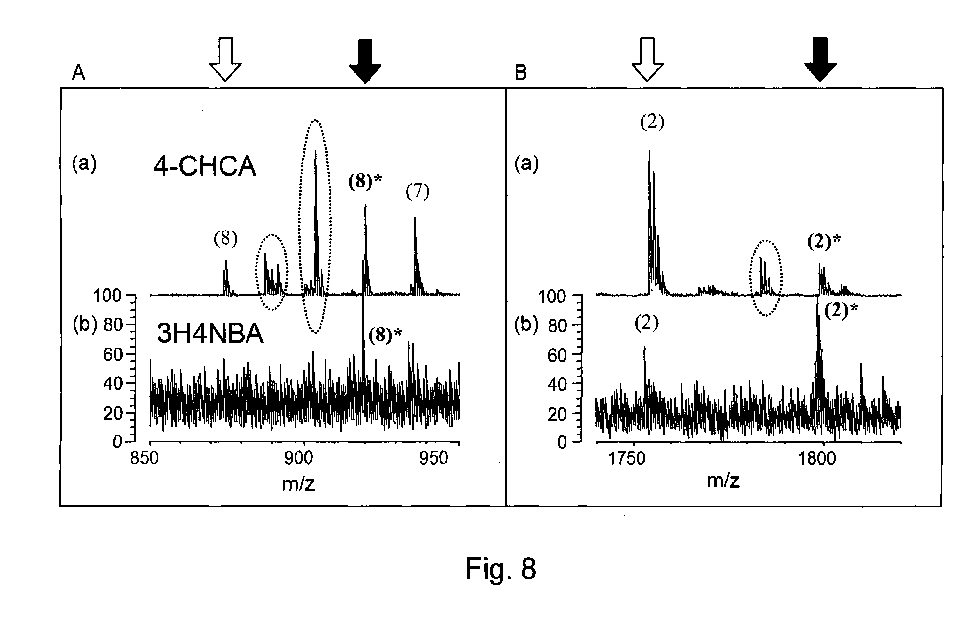

Fig. 8 is a partially enlarged view of Fig. 7;

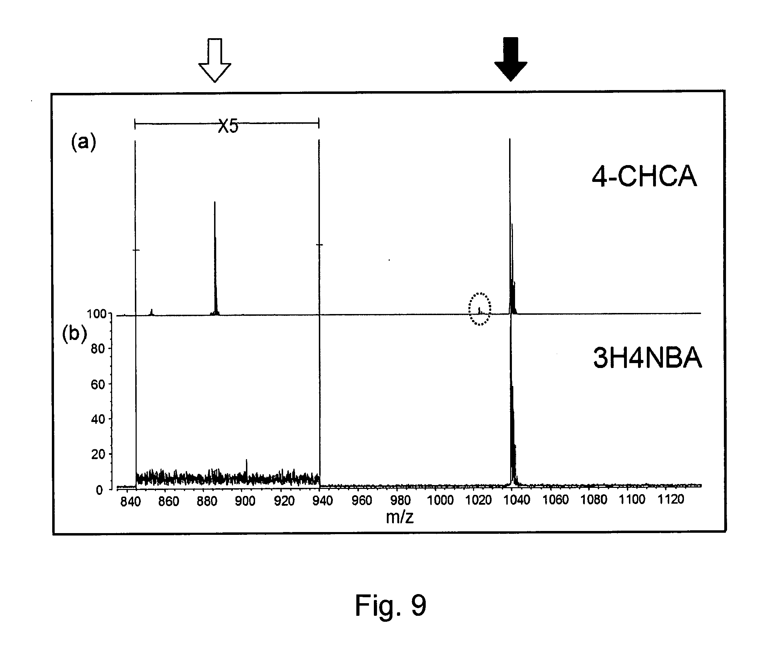

Fig. 9 shows a mass spectrum (a) obtained by the conventional method and a mass spectrum (b) obtained by the present invention when the sample to be measured is a mixture of peptide IRRPl modified with NBS Reagent (light) and unmodified peptide IRRPl;

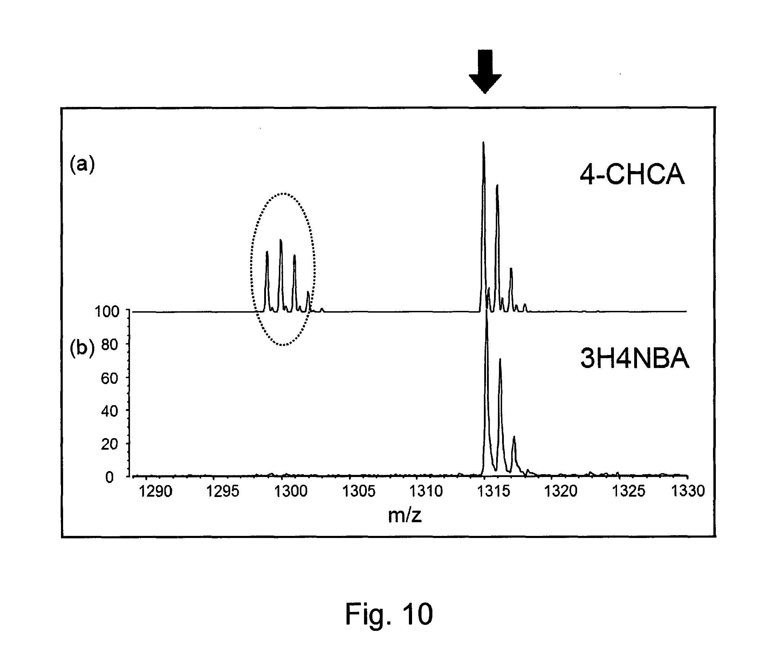

Fig. 10 includes a mass spectrum (a) obtained by the conventional method and a mass spectrum (b) obtained by the present invention when the sample to be measured is peptide HIV subIII containing *p*-nitrophenylalanine;

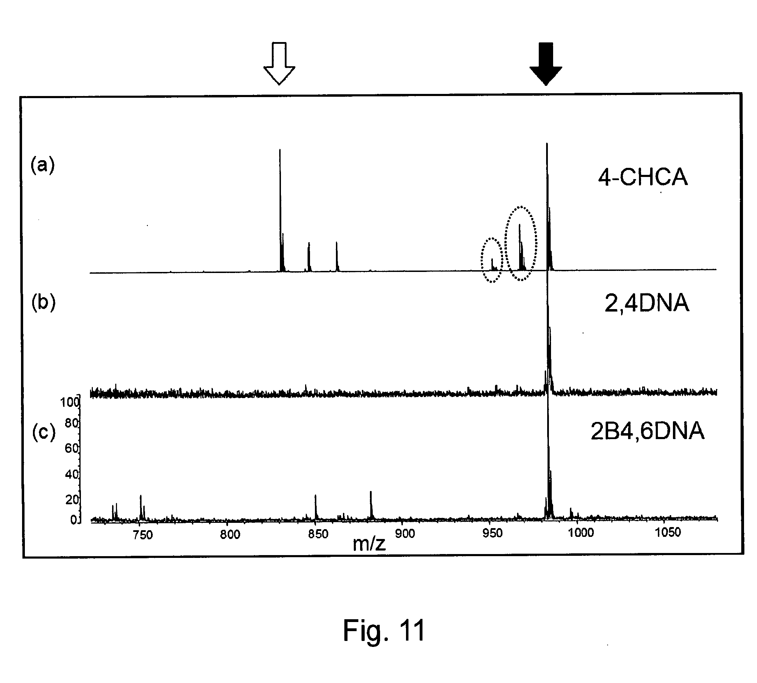

Fig. 11 includes a mass spectrum (a) obtained by the conventional method and mass spectra ((b) and (c)) obtained by the present invention when the sample to be measured is ACTH modified with NBS Reagent (light) and unmodified ACTH;

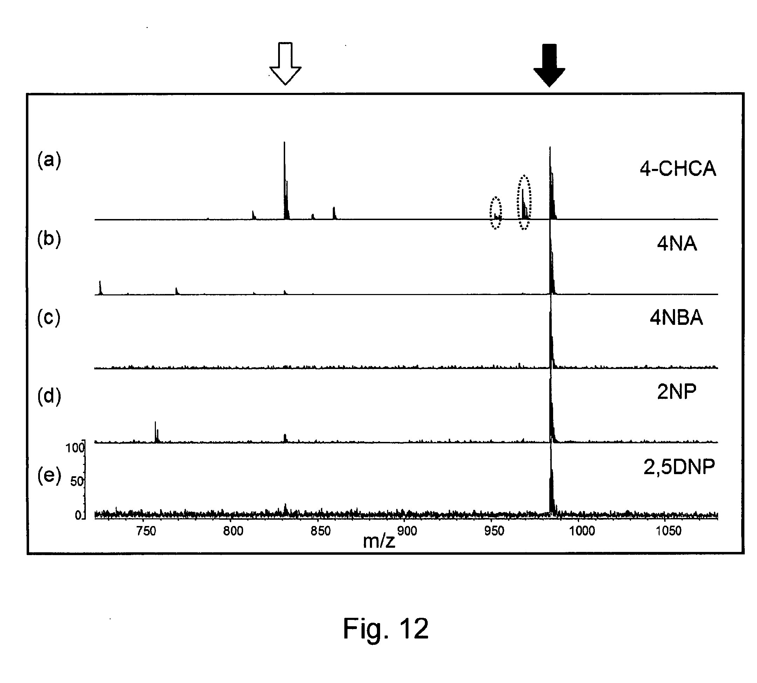

Fig. 12 includes a mass spectrum (a) obtained by the conventional method and mass spectra ((b), (c), (d) and (e)) obtained by the present invention when the sample to be measured is ACTH modified with NBS Reagent (light) and unmodified ACTH; and

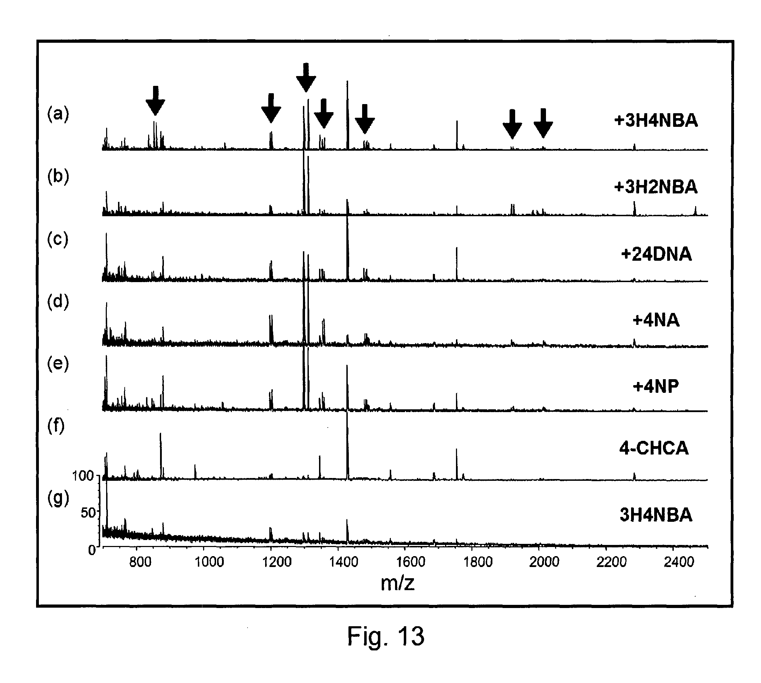

Fig. 13 shows a mass spectrum (f) obtained by the conventional method and mass spectra ((a), (b), (c), (d), (e) and (g)) obtained by the present invention when the samples to be measured are prepared by digesting a 1:1 mixture of NBS Reagent (heavy) modified- and NBS Reagent (light) modified- four purified proteins (ovalbumin, glyceraldehyde-3-phosphate dehydrogenase, lysozyme, and α-lactalbumin).

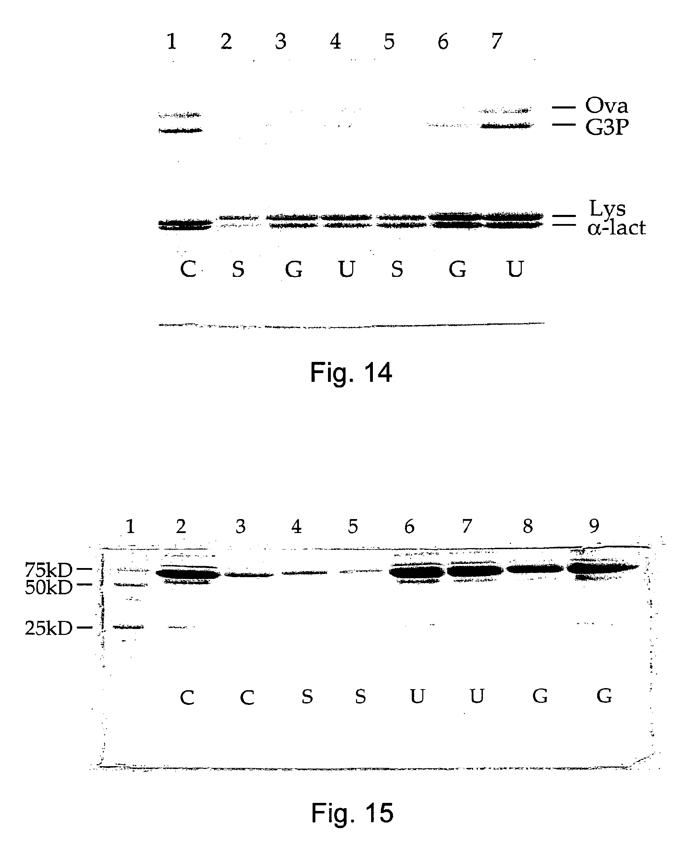

Fig. 14 to Fig. 23 relate to the third invention.
   Fig. 14 is an electrophoretic diagram obtained in Experimental Example C-1;
Fig. 15 is an electrophoretic diagram obtained in Experimental Example C-2;

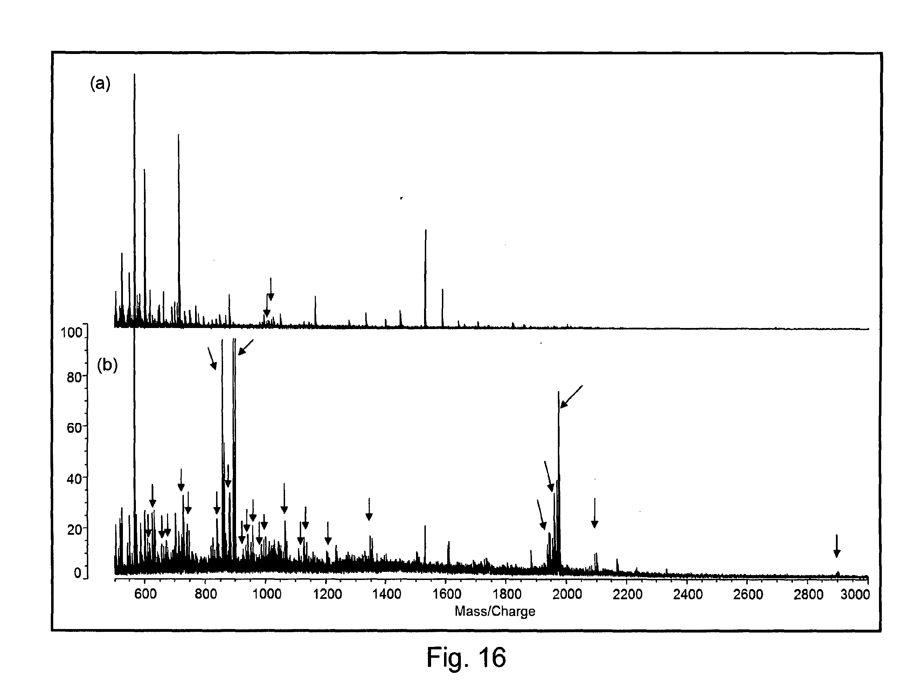

Fig. 16 is MS spectra obtained in Experimental Example C-3;

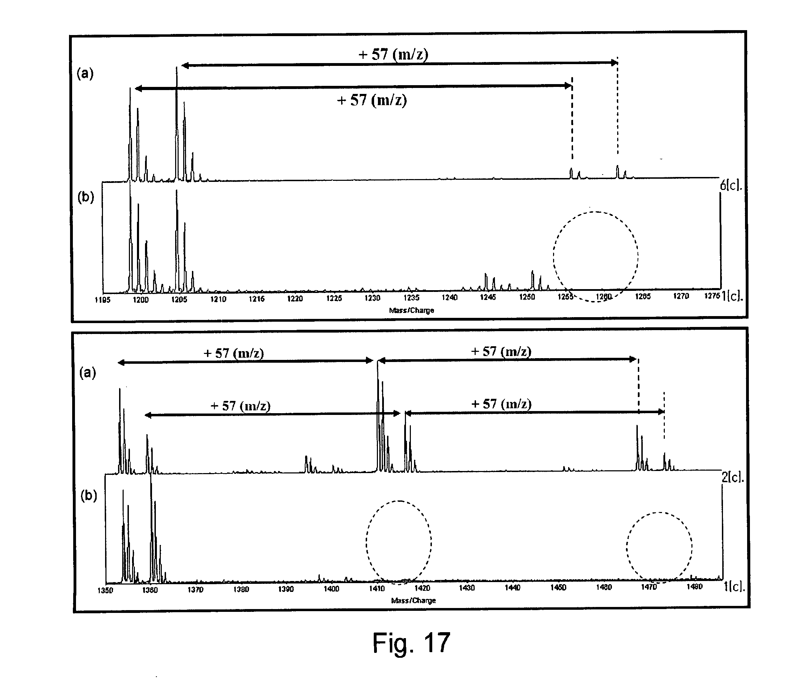

Fig. 17 is MS spectra obtained in Experimental Example C-5;

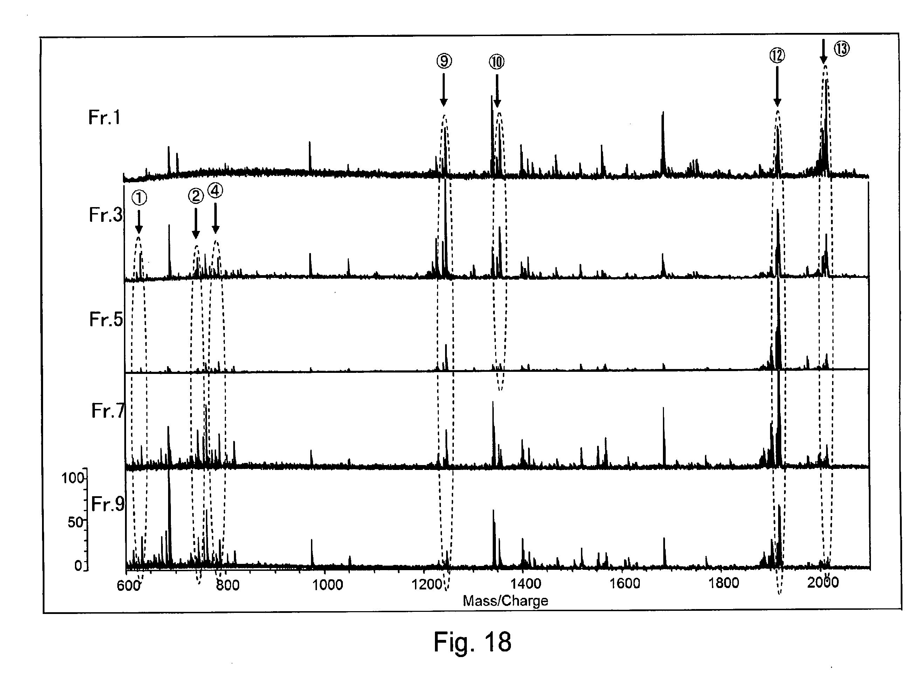

Fig. 18 is MS spectra obtained in Experimental Example C-6;

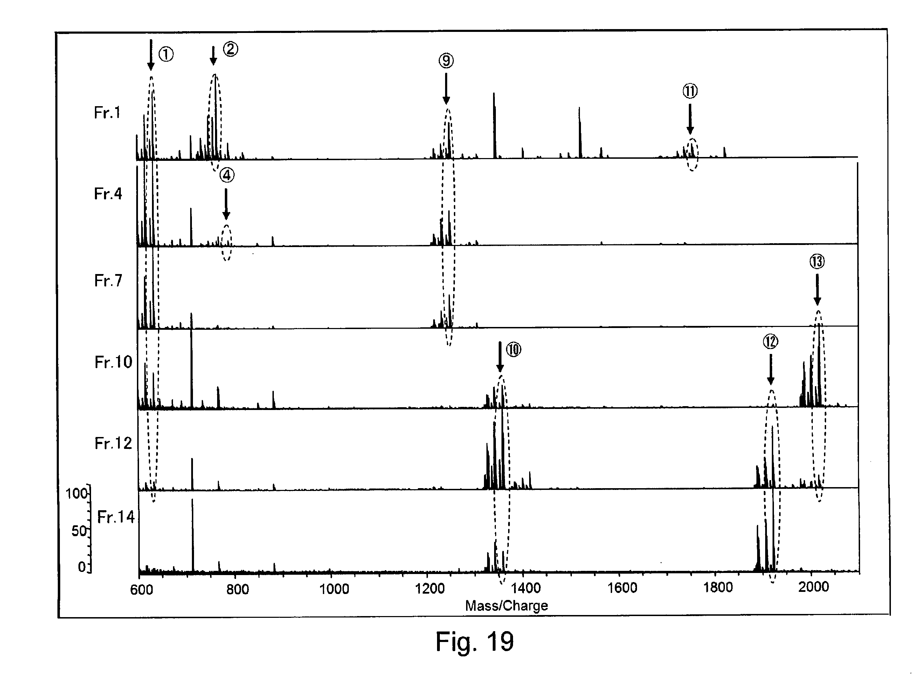

Fig. 19 is MS spectra obtained in Experimental Example C-6;

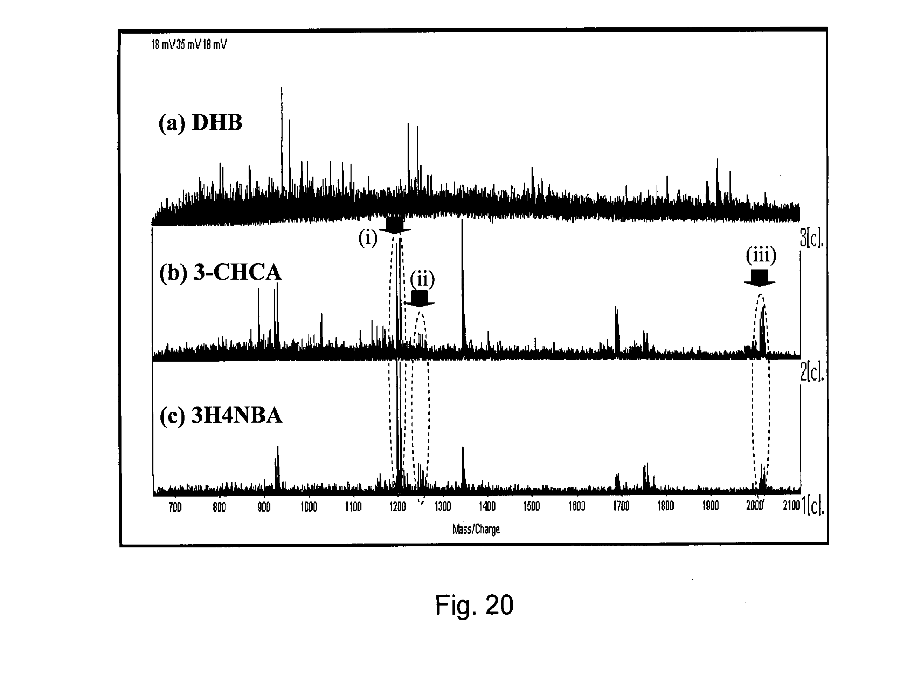

Fig. 20 is MS/MS spectra obtained in Experimental Example C-7;

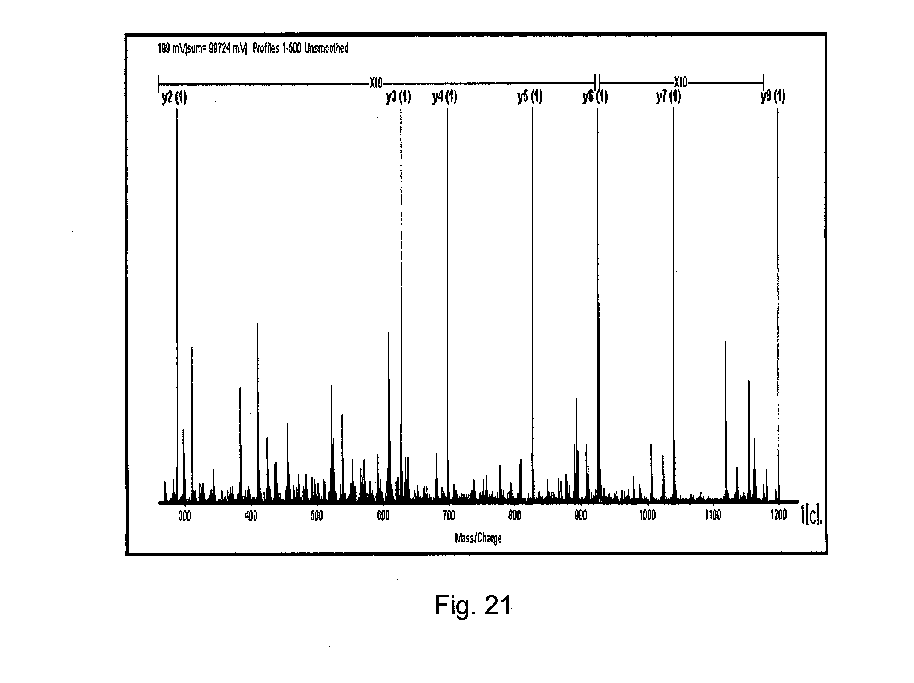

Fig. 21 is a MS spectrum obtained in Experimental Example C-7;

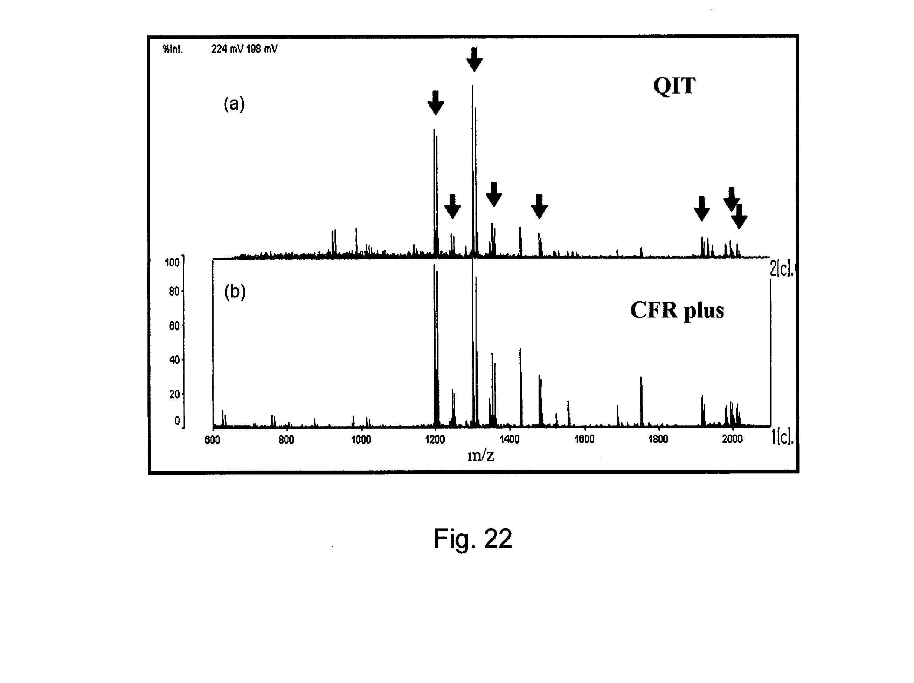

Fig. 22 is MS spectra obtained in Experimental Example C-8; and

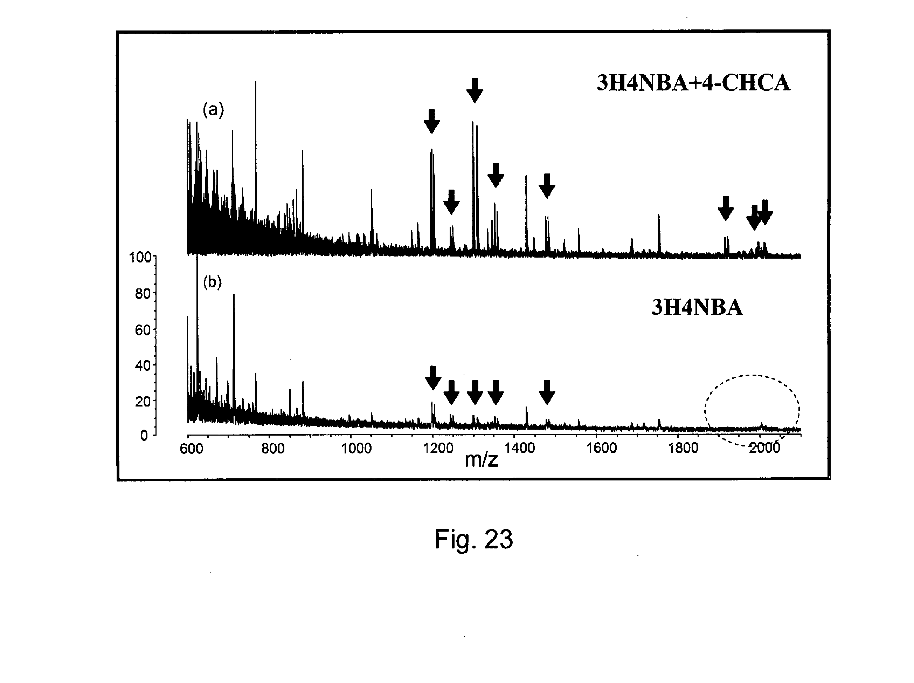

Fig. 23 is MS spectra obtained in Experimental Example C-9.

### MODES FOR CARRYING OUT THE INVENTION

Modes for carrying out the first invention will be described as follows.

The present method is a method of measuring a hydrophobic peptide analyte using a mass spectrometer having a MALDI (Matrix Assisted Laser Desorption/Ionization) ion source. Ionization mechanism of the MALDI method is not completely elucidated at this time. Description of the present invention is based on the interpretation that is most widely accepted in the art.

In the present invention, the term "hydrophobic peptide" or "hydrophobic protein" refers to those having relatively high contents of hydrophobic amino acids, especially highly-hydrophobic amino acids, of the amino acid composition of such peptide or protein; those having the groups exemplified below; and those having chemical modifications as described below. Examples of the highly-hydrophobic amino acids include tryptophan, isoleucine, tyrosine, phenylalanine, leucine, valine, and methionine. Alanine, glycine, proline, and the like are also considered as hydrophobic amino acids in some cases.

The present invention is especially useful in measuring a hydrophobic peptide having a nitrobenzenesulfenyl group (NBS group: NO₂PhS group in which Ph represents a phenylene group), a nitrophenyl group, or the like. Examples of such a hydrophobic peptide include peptides containing a nitrotyrosine residue, a nitrophenylalanin residue, a tryptophan residue having a NBS group as a substituent, or a cysteine residue having a NBS group as a substituent.

The hydrophobic peptide may be obtained by chemically modifying a corresponding peptide with a hydrophobic compound. The corresponding peptide may itself be hydrophobic. In other words, the hydrophobic peptide may be derivatized into a peptide having higher hydrophobicity through chemical modification using a hydrophobic compound. Preferably, the hydrophobic compound is a sulpheyl compound. Sulfenyl compound is a compound that specifically modifies a tryptophan reside and a cysteine residue in a peptide or a protein. Particularly preferred examples of the sulfenyl compound include 2-nitrobenenesulfenyl chloride (NBS reagent). That is, an example of the hydrophobic peptide preferred in the present invention is those obtained by chemically modifying a peptide having a tryptophan residue or a cysteine residue using an NBS reagent.

The method of the present invention is particularly useful to mass spectrometers, combined with a MALDI ion source, in which the time required for a generated ion to reach a detector and undergo measurement is longer than that of a commonly-used MALDI-TOF mass spectrometer. Examples of such mass spectrometers include a MALDI-IT (Matrix Assisted Laser Desorption/Ionization - Ion Trap) mass spectrometer; a MALDI-IT-TOF (Matrix Assisted Laser Desorption/Ionization - Ion Trap - Time of Flight) mass spectrometer; and a MALDI-FTICR (Matrix Assisted Laser Desorption/Ionization - Fourier Transform Ion Cyclotron Resonance) mass spectrometer.

In the present invention, α-cyano-3-hydroxycinnamic acid (3-CHCA: Formula A-I below) or 3-hydroxy-4-nitrobenzoic acid (3H4NBA: Formula A-II below) is used as a matrix.

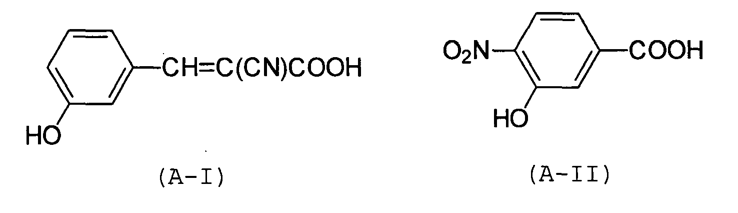

A person skilled in the art may appropriately determine the use form of these compounds in view of the use as a matrix for mass spectrometry. For example, these compounds are preferably used in a solution state. The concentration of the solution is not limited, for example, the solution may be used at a concentration of 1 mg/ml to a saturated concentration.

Preferred solvents used in preparing the above solution include an aqueous solution of acetonitrile, an aqueous solution of trifluoroacetic acid (TFA), or an aqueous solution of acetonitrile-trifluoroacetic acid (TFA). When the aqueous solution of acetonitrile or the aqueous solution of acetonitrile-TFA is used, the concentration of acetonitrile may be, but not limited to, not more than 90%, preferably about 50%. When the aqueous solution of TFA or the aqueous solution of acetonitrile-TFA is used, the concentration of TFA may be, but not limited to, not more than 1%, preferably about 0.1%.

When 3-CHCA is used as a matrix, 3-CHCA is dissolved in the above solvent and may be used as a matrix solution having a concentration of, for example, but not limited to, 1 mg/ml to a saturated concentration, preferably 10 mg/ml. When 3H4NBA is used as a matrix, 3H4NBA is dissolved in the above solvent and may be used as a matrix solution having a concentration of, for example, but not limited to, 1 mg/ml to a saturated concentration, preferably a saturated concentration.

The amount expressed as % in this description is on the basis of v/v% unless otherwise specified.

By using the matrix as described above, the following advantages are achieved.

As described above, in MALDI-IT mass spectrometer, MALDI-IT-TOF mass spectrometer, MALDI-FTICR mass spectrometer, and the like, the time required for a generated ion to reach the detector and undergo measurement is longer than that in a commonly-used MALDI-TOF mass spectrometer. In addition, the matrix is heated by receiving energy of a laser to be vaporized and ionized, and the energy partially becomes internal energy of the analyte molecules to cause gradual self-disintegration of ions. In other words, the longer the time required for a generated ion to reach the detector and undergo measurement, the more the self-disintegration proceeds, resulting that a number of undesired fragment ions will be detected. The matrix used in the present invention is believed to give smaller internal energy to the molecules to be measured during ionization, compared to the matrix in conventional methods (for example, the method in which only 4-CHCA is used as a matrix). Therefore, even if it is used in a mass spectrometer in which a generated ion requires a long time to reach the detector and undergo measurement as described above, it is possible to suppress the progression of self-disintegration of generated ions.

For this reason, the present invention is especially useful in a mass spectrometer in which a time from ionization to detection of ion is relatively long such as an ion trap mass spectrometer. Therefore, it goes without saying that it is also useful in a mass spectrometer having no ion trap.

Furthermore, as is already mentioned, affinity between matrix and analyte molecule is an important factor in ionization. It is expected that a matrix having high hydrophobicity is well compatible with an analyte having high hydrophobicity, and a matrix having high hydrophilicity is well compatible with an analyte having high hydrophilicity. It is expected that 3-CHCA or 3H4NBA matrix used in the present invention has higher hydrophobicity than conventional matrixes (DHB, for example). On the other hand, it is expected that peptide analyte molecules to be measured in the present invention also have high hydrophobicity, so that they have high affinity with 3-CHCA or 3H4NBA as a matrix. As a result, it is expected that a peptide analyte and a matrix is easy to form uniform fine crystals, and effective ionization can be achieved.

In the present invention, when 3-hydroxy-4-nitrobenzoic acid (3H4NBA) is used as a matrix, 3H4NBA is preferably used as a mixed matrix in which 3H4NBA is combined with α-cyano-4-hydroxycinnamic acid (4-CHCA, Formula A-III below). 4-CHCA is a compound widely used as a matrix in mass spectrometric analysis of a peptide. (Hereinafter, a singularly used matrix in which the matrix is used without combining with 4-CHCA, and a mixed matrix in which 4-CHCA is used in combination are sometimes simply described as matrix.)

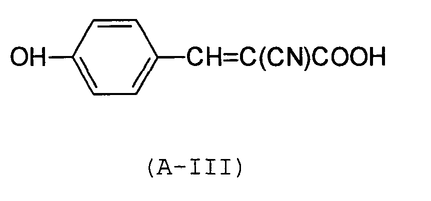

3H4NBA and 4-CHCA may be combined, for example, in the following quantitative relationship in a nonrestrictive manner.

For example, 3H4NBA may be prepared at a concentration as described above. Specifically, a 3H4NBA solution may be prepared as an aqueous solution having a concentration of 1 mg/ml to a saturated concentration; for example, when an aqueous solution of acetonitrile, an aqueous solution of TFA, or an aqueous solution of acetonitrile-TFA is used as a solvent, a 3H4NBA solution may be prepared as a solution having a concentration of 1 mg/ml to a saturated concentration, preferably a saturated concentration.

On the other hand, 4-CHCA may be prepared in such a concentration that is conventionally used. For example, it may be prepared as a solution having a concentration of 1 mg/ml to a saturated concentration. When an aqueous solution of acetonitrile, an aqueous solution of TFA, or an aqueous solution of acetonitrile-TFA same as used in preparing the 3H4NBA solution is used as a solvent, a 4-CHCA solution may be prepared as a solution having a concentration of 1 mg/ml to a saturated concentration, preferably 10 mg/ml.

The both of solution prepared in these manners are mixed in a volume ratio of, preferably 1:10 to 10:1, more preferably 1:3 to 3:1, for example 1:1 for use.

The conventional matrix 4-CHCA has a drawback that self-disintegration of an analyte to be measured occurs during measurement with a MALDI spectrometer such as MALDI-IT, MALDI-IT-TOF, or MALDI-FTICR spectrometer in which a time from ionization to detection of ion is long. However, the conventional matrix 4-CHCA shows excellent measuring sensitivity and is advantageous in that an optimum spot on which a laser is to be focused can be easily found in a mass spectrometric sample.

On the other hand, the matrix 3H4NBA of the present invention can advantageously suppress the progression of self-disintegration of an analyte to be measured, and achieve efficient ionization of a hydrophobic analyte. The matrix 3H4NBA of the present invention is used in combination with 4-CHCA, thereby synergistic effect of the advantages given by both of the matrixes is exerted. In brief, an ability to detect with high sensitivity possessed by 4-CHCA is added while maintaining the advantages that 3H4NBA by itself have: suppression of self-disintegration of an analyte to be measured, and achievability of ionization of hydrophobic analyte, and it is possible to conduct a mass spectrometry with higher analytical efficiency.

According to the present invention, the applicable range of method capable of determining a sequence of a hydrophobic peptide (for example, NBS labeled peptide) is significantly increased. In a conventional technique, a MS/MS analysis by a mass spectrometer using an ESI (Electrospray Ionization) and a PSD analysis using a MALDI mass spectrometer were used. The present invention newly enabled a MS/MS analysis by a MALDI-IT mass spectrometer, a MALDI-IT-TOF mass spectrometer, a MALDI-FTICR mass spectrometer, and the like which can cause CID (collision induced dissociation). In addition, since CID can cause fragmentation more effectively than PSD, it became possible to improve the identification rate of peptides.

Modes for carrying out the second invention will be described as follows.

In the following, the present invention will be described in detail. The mechanism of ionization in the MALDI method is not completely elucidated. The concept described in the present specification is based on the understandings predominantly supported in the art, however it still remains in the realm of speculation.

A sample to be subjected to mass spectrometry is a mixture containing a specific substance to be measured and a substance other than the specific substance. A matrix which is more likely to ionize the specific substance to be measured than the substance other than the specific substance is used. Preferably, a matrix that mainly ionizes the specific substance to be measured, but hardly ionizes the substance other than the specific substance is used (hereinafter, the substance other than the specific substance is referred to as "other substances" in some cases).

In the MALDI method, the analyte molecule and the matrix are vaporized and ionized almost simultaneously. In other words, the matrix assists ionization of the analyte molecule while dispersing the analyte molecule at the molecular level. In order to vaporize and ionize efficiently, affinity between the matrix and the analyte molecule would be important. Higher affinity between the matrix and the analyte molecule will be easy of uniform dispersion of the analyte molecule in the matrix at the molecular level, or easy of uniform formation of mixed crystal.

Therefore, from the view point of the relationship between ionization and affinity, in the present invention, preferably, the matrix has a structure having affinity with the specific substance, the specific substance has a structure having affinity with the matrix, and other substances do not have such a structure. In the present invention, combination of the specific substance and the matrix from the view point of affinity therebetween allows specific ionization of the specific substance.

Examples of the specific substance include, but not limited to, substances related to a living organism such as proteins, peptides, sugars, lipids, and the like. In contrast to the specific substance, the other substance than the specific substance is not particularly limited insofar as they do not have the structure having affinity with the matrix. Generally, in the present invention, when the specific substance is a substance related to a living organism, the other substance is also a living organism-related substance belonging to a similar category with the specific substance insofar as it lacks the structure as described above. For example, when the specific substance is a peptide, the other substance is often a peptide not having the aforementioned structure. In the present invention, the other substance may include substances existing with and byproducts in the course of preparing the specific substance.

The terms proteins, peptides, sugars, and lipids used herein include modified compounds thereof. The modified compounds also include chemically-modified compounds. When the modified compound corresponds to the specific substance, the modified compound has a group having a structure that has affinity with the matrix, and the chemically-modified compound is obtained by chemically-modifying with the group having the aforementioned structure.

The specific substance may be isotope-labeled. In this case, the specific substance may be a mixture of a labeled substance and an unlabeled substance corresponding thereto.

One example of a sample mixture that is an object of mass spectrometry is a mixture of modified peptide and unmodified peptide as is obtained in the NBS method described in the foregoing conventional art. In this sample mixture, a modified peptide is selectively detected as a specific substance. As described above, in the NBS method, the modified peptide selected as a specific substance to be detected is basically a mixture of peptide modified with a stable isotope (¹³C)-labeled compound and peptide modified with an unlabeled compound. In the present invention, by using a matrix that predominantly ionizes modified peptides and hardly ionizes unmodified peptides, it is possible to specifically ionize the modified peptides. In this way, by using the present invention, it is possible to selectively detect modified peptides in mass spectrometry even in a mixture state without separating the modified peptides and the unmodified peptides.

As is already mentioned above, the present invention utilizes affinity between a matrix and an analyte molecule. As an interaction that leads such affinity, van der Waals interaction, π-π electrons interaction, hydrophilic interaction, hydrophobic interaction, or the like is used. Depending on the sample to be measured, a substance that is able to interact with the specific substance in the sample through the above interaction is selected as a matrix. For example, when the specific substance is a π electron containing compound, a n electron containing compound may be selected for the matrix to utilize the π-π electrons interaction therebetween. When the specific substance is a hydrophobic compound, a hydrophobic compound may be selected for the matrix to utilize the hydrophobic interaction therebetween. When the specific substance is a hydrophilic compound, a hydrophilic compound may be selected for the matrix to utilize the hydrophilic interaction therebetween. Preferably the kind of the specific substance to be measured and the kind of the matrix is selected so that the above interactions are combined. The matrix thus selected would homogenously mingles with the specific substance through the above interaction, or forms uniform mixed crystal, enabling predominant vaporization and ionization of the specific substance as well as its own vaporization and ionization.

Now the specific substance to be measured will be explained when the specific substance to be measured is a hydrophobic peptide or a hydrophobic protein. In the present invention, the term "hydrophobic peptide" or "hydrophobic protein" refers to those having relatively high contents of hydrophobic amino acids, especially highly-hydrophobic amino acids, of the amino acid composition of such peptide or protein; those having the groups exemplified below; and those having chemical modifications as described below. Examples of the highly-hydrophobic amino acids include tryptophan, isoleucine, tyrosine, phenylalanine, leucine, valine, and methionine. Alanine, glycine, proline, and the like are also considered as hydrophobic amino acids in some cases. Further, the hydrophobic peptide or hydrophobic protein preferably has a benzene ring and/or an aromatic ring other than benzene ring, and preferably has a nitro group.

The present invention is usefully applied, in particular, to a hydrophobic peptide or hydrophobic protein having a benzene ring and/or aromatic ring other than benzene ring which has a nitro group as a substituent. More specifically, the hydrophobic peptide or hydrophobic protein more preferably has a nitrobenzenesulfenyl group (NBS group: NO₂PhS in which Ph represents a phenylene group) or nitrophenyl group. Protein or peptide containing a nitrotyrosine residue, a nitrophenylalanine residue, a tryptophan residue having a NBS group as a substituent, or a cysteine residue having a NBS group as a substituent is exemplified.

The hydrophobic peptide or hydrophobic protein may be obtained by chemically modifying a corresponding peptide or protein with a hydrophobic compound. This aspect encompasses the case where the peptide or protein corresponding to the hydrophobic peptide or the hydrophobic protein itself is hydrophobic. In other words, this aspect encompasses the case where a hydrophobic peptide or a hydrophobic protein is derived into a peptide or protein having higher hydrophobicity through chemical modification using a hydrophobic compound. As the hydrophobic compound, hydrophobic compounds having a benzene ring, an aromatic ring other than benzene ring and/or a nitro group may be exemplified. In the present invention, the hydrophobic compound is preferably a sulfenyl compound. "Sulphenyl compound" is a compound that specifically modifies a tryptophan residue and a cysteine residue in a peptide or a protein. Particularly preferred examples of the sulfenyl compound include 2-nitrobenenesulfenyl chloride (NBS reagent). That is, an example of the hydrophobic peptide or hydrophobic protein preferred in the present invention is those obtained by chemically modifying a peptide or protein having a tryptophan residue or a cysteine residue using a NBS reagent.

The hydrophobic peptide or hydrophobic protein may be obtained by nitrating a corresponding peptide or protein. As is already mentioned, the corresponding peptide or protein may itself be hydrophobic. Another example of a preferred hydrophobic peptide or hydrophobic protein in the present invention is those obtained by nitration by an electrophilic aromatic substitution of a peptide or protein having a benzene ring and/or an aromatic ring other than benzene ring using a conventionally used method.

A preferred matrix used together with such a hydrophobic peptide or a hydrophobic protein is a substituted aromatic compound having a functional group for transferring electric charges from/to the specific substance to be measured and a functional group for affording hydrophobicity to the matrix molecule itself. As the substituted aromatic compound, a substituted benzene compound is preferred. A functional group for transferring electric charge is a functional group for transferring an electron or a proton, and examples of such a functional group include carboxyl group, hydroxyl group, amino group, sulfate group, nitrate group and aldehyde group. As a functional group for affording hydrophobicity, a nitro group is exemplified.

Among these substituted aromatic compounds, a nitrobenzoic acid derivative, a nitrophenol derivative and a hydroxynitrobenzoic acid derivative are preferred in the present invention. In particular, those selected from ten aromatic positional isomers of hydroxynitrobenzoic acid (HNBA) are preferred.

Concrete examples of the present invention include 4-nitroaniline (4NA: Formula (B-I) below), 2,4-dinitroaniline (2,4DNA: Formula (B-II) below), 2-bromo-4,6-dinitroaniline (2B4,6DNA: Formula (B-III) below), 4-nitrophenol (4NP: Formula (B-IV) below), 2-nitrophenol (2NP: Formula (B-V) below), 2,5-dinitrophenol (2,5DNP:
Formula (B-VI) below), 4-nitrobenzoic acid (4NBA: Formula (B-VII) below), 3-hydroxy-4-nitrobenzoic acid (3H4NBA: Formula (B-VIII) below), and 3-hydroxy-2-nitrobenzoic acid (3H2NBA: Formula (B-IX) below). Structural formulas of these compounds are shown below.

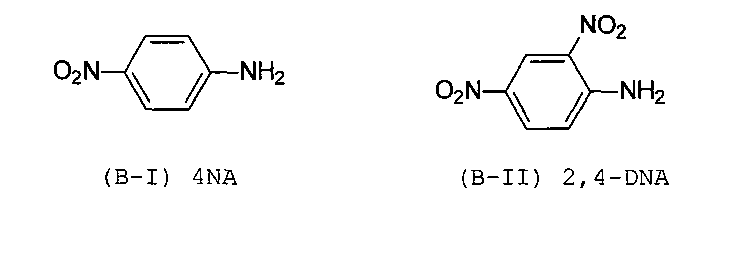

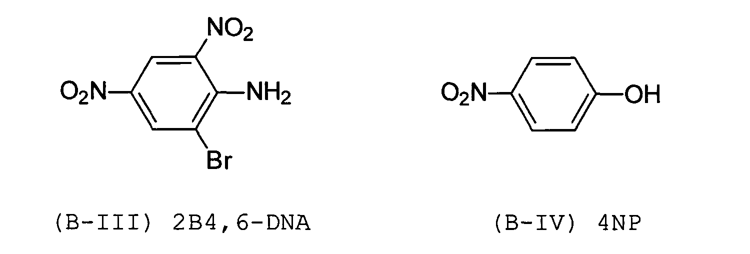

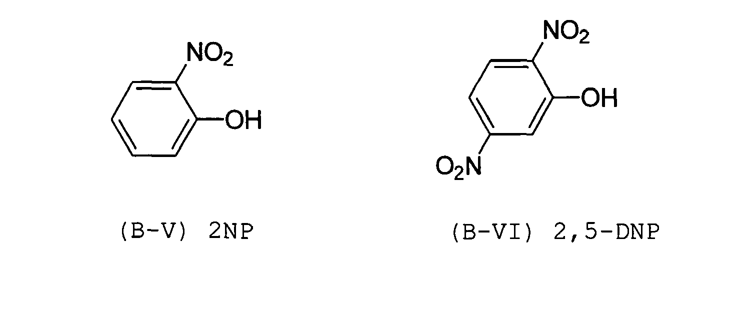

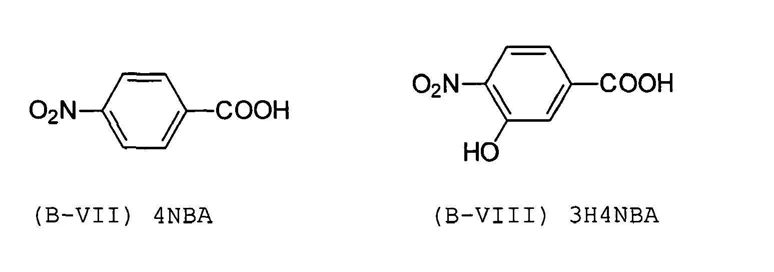

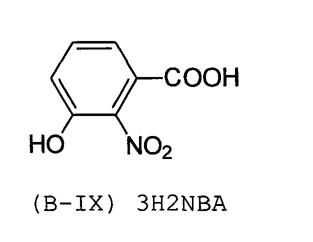

A person skilled in the art may appropriately determine the use form of these compounds in view of the use as a matrix for mass spectrometry. For example, these compounds are preferably used in a solution state. For example, the solution may be used at a concentration of 1 mg/ml to a saturated concentration.

Preferred solvents used in preparing the above solution include an aqueous solution of acetonitrile, an aqueous solution of trifluoroacetic acid (TFA), or an aqueous solution of acetonitrile-trifluoroacetic acid (TFA). When the aqueous solution of acetonitrile or the aqueous solution of acetonitrile-TFA is used, the concentration of acetonitrile may be, but not limited to, not more than 90%, preferably about 50%. When the aqueous solution of TFA or the aqueous solution of acetonitrile-TFA is used, the concentration of TFA may be, but not limited to, not more than 1%, preferably about 0.1%.

2,4-dinitroaniline, 2-bromo-4,6-dinitroaniline, 3-hydroxy-4-nitrobenzoic acid, 4-nitrobenzoic acid, and 3-hydroxy-4-nitrobenzoic acid may be dissolved in the above solvent and may be used as a matrix solution having a concentration of, for example, but not limited to, 1 mg/ml to a saturated concentration, preferably 10 mg/ml. 4-nitroaniline, 4-nitrophenol, 2-nitrophenol, 2,5-dinitrophenol, and 3-hydroxy-2-nitrobenzoic acid may be dissolved in the above solvent and may be used as a matrix solution having a concentration of, for example, but not limited to 1 mg/ml to a saturated concentration, preferably a saturated concentration.

The amount expressed as % in this description is on the basis of v/v% unless otherwise specified.

In the present invention, especially preferred combination of the specific substance to be measured and the matrix is a peptide or protein modified with NBS reagent and a substituted aromatic compound having a nitro group. Both of the specific substance to be measured and the matrix recited above have a nitrobenzene structure.

As is already mentioned, in the MALDI method, the analyte and the matrix are vaporized and ionized almost simultaneously. Accordingly, affinity between the analyte and the matrix that is required for forming uniform mixed crystal is thought to be related in detection of the analyte. In a specific detection of NBS-modified peptide or protein using a substituted aromatic compound having a nitro group as a matrix, the combination of the analyte and the matrix is thought to be related with existence of a nitro group, as well as with affinity caused by hydrophobic interaction and π-π electrons interaction.

Since the specific sample to be measured and the matrix are combined in a preferable way, the present invention is applied especially usefully to a sample to be measured which is not subjected to a treatment such as enrichment/separation. Enrichment/separation is sometimes difficult or inadequate, and this may lead increase in handling processes up to measurement and concomitant increase in loss of sample, time, cost and the like. Hence, the method of the present invention is very convenient and cost-effective. It goes without saying that the present invention that enables specific detection with high sensitivity can be usefully applied to a sample to be measured having subjected to a treatment such as enrichment/separation.

In the present invention, in addition to the use of each compounds recited above by itself as a matrix, a mixed matrix as follows is also preferably used.

A mixed matrix of the present invention is a mixture of the above matrix and α-cyano-4-hydroxycinnamic acid (4-CHCA, Formula (B-X) below). α-cyano-4-hydroxycinnamic acid is a compound widely used as a matrix in mass spectrometric analysis of a peptide. (Hereinafter, a singularly used matrix in which it is used without combining with 4-CHCA, and a mixed matrix in which 4-CHCA is used in combination are sometimes collectively described as matrix.)

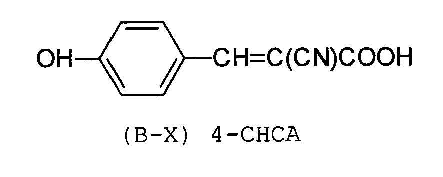

The matrix and 4-CHCA may be combined, for example, in the following quantitative relationship in nonrestrictive manner.

The matrix may be prepared in such an amount as described above. That is, the matrix may be prepared as a solution of 1 mg/ml to saturated concentration. For example, when an aqueous solution of acetonitrile, an aqueous solution of TFA, or an aqueous solution of acetonitrile-TFA is used as a solvent, 2,4-dinitroaniline, 2-bromo-4,6-dinitroaniline, 3-hydroxy-4-nitrobenzoic acid, 4-nitrobenzoic acid, and 3-hydroxy-4-nitrobenzoic acid may be prepared as a matrix solution of 1 mg/ml to a saturated concentration, preferably a saturated concentration. For example, when the above described aqueous solution is used as a solvent, 4-nitroaniline, 4-nitrophenol, 2-nitrophenol, 2,5-dinitrophenol and 3-hydroxy-2-nitrobenzoic acid may be prepared as a matrix solution of 1 mg/ml to a saturated concentration, preferably 10 mg/ml.

On the other hand, 4-CHCA may be prepared as a solution having a concentration of 1 mg/ml to a saturated concentration. When an aqueous solution of acetonitrile, an aqueous solution of TFA or an aqueous solution of acetonitrile-TFA same as used in preparing the above matrix solution is used as a solvent, a 4-CHCA solution having a concentration of 1 mg/ml to a saturated concentration, preferably 10 mg/ml may be prepared.

The both of the solutions prepared in the above manners are mixed in a volume ratio of, preferably 1:10 to 10:1, more preferably 1:3 to 3:1, for example 1:1 for use.

The conventional matrix 4-CHCA can not necessarily detect a specific substance in a specific manner in mass spectrometry measurement of a sample to be measured not subjected to enrichment procedure. However, the matrix 4-CHCA is excellent in measuring sensitivity and has an advantage that an optimal spot on which a laser beam is focused in a mass spectrometric sample can be readily found.

On the other hand, matrixes of the present invention such as nitrobenzene derivatives, for example, 4-nitroaniline, 2,4-dinitroaniline, 2-bromo-4,6-dinitroaniline, 4-nitrophenol, 2-nitrophenol, 2,5-dinitrophenol, 4-nitrobenzoic acid, 3-hydroxy-4-nitrobenzoic acid and 3-hydroxy-2-nitrobenzoic acid are, as is described above, believed to have excellent affinity with a specific substance in a sample to be measured of the present invention, and therefore have an advantage that an efficient ionization of the specific substance can be achieved. Further, when these matrices are used in combination with 4-CHCA as a mixed matrix, synergistic effect of the advantages given by each matrix is exerted. In brief, an ability of 4-CHCA to detect with high sensitivity is added while not spoiling at a practical level the specific detectability that each of these matrices has by itself. Therefore, it is possible to conduct mass spectrometry with higher analytical efficiency.

According to the present invention, it is possible to selectively detect an objective substance. The present invention is used usefully, especially in the above NBS method, when an objective NBS-modified peptide is detected from a mixed sample containing NBS-modified peptides and unmodified peptides. Basically, in this case, pairs of peaks derived from a peptide modified with 2-nitro[¹³C₆] benzenesulfenyl chloride and a peptide modified with 2-nitro [¹²C₆] benzenesulfenyl chloride are specifically detected.

According to the present invention, it is possible to readily recognize a protein contained in either one of a sample to be analyzed and a reference sample. For example, when the present method is used in the step (6) of the aforementioned NBS method, a protein contained in either one of the samples can be recognized as follows. A mass spectrometric sample obtained by conducting the foregoing step (5) includes modified peptides derived from protein contained in both of the protein sample I and protein sample II (referred to as A, A', respectively), and modified peptide from protein contained in either one of the samples (referred to as B). The mass spectrometric sample further includes unmodified peptide that failed to be removed because of inadequate enrichment/ separation (referred to as C). When the conventional matrix (for example, 4-CHCA) is used and mass spectrometry is conducted, A and A' are detected as a pair of peaks, while peptides B and C are detected as single peaks, and whether B and C are modified or unmodified is not distinguished. Contrarily, when the matrix of the present invention is used, the modified A, A' and B are predominantly detected, while C is hardly detected. Among these predominantly detected peaks, the pair of peaks are modified peptides A and A' derived from proteins contained in both of the samples, and the single peak is a modified peptide B derived from a protein contained in either one of the samples. In brief, among the single peaks detected in a result of mass spectrometry using the conventional matrix, the distinction can be made that: "B" which is also detected in the mass spectrometry using a matrix of the present invention is a labeled peptide derived from a protein contained in either one of the samples, and "C" which is hardly detected is an unmodified peptide.

When the NBS-modified peptide is analyzed using 4-CHCA as a matrix as is the conventional method, a peak is observed also at a position smaller by m/z value of 16 than that of the peak for the objective peptide. Also a minor peak at a position smaller by m/z value of 32 than that of the peak for the objective peptide is sometimes observed. Depending on the peptide to be measured, also a peak at a position smaller by m/z value of 155 than that of the peak for the objective peptide is observed (when modified with NBSCl (light) available from SHIMADZU). By the use of the present invention, these peaks are hardly observed and mass spectrum is simplified, which facilitates the analysis.

Modes for carrying out the third invention will be described as follows.

The method of the present invention includes sample preparation step (i); solubilization step (ii); modification step (iii); desalting step (iv); resolubilization step (v); reduction and alkylation step (vi); digestion step (vii); enrichment/separation step (viii); and mass spectrometry step (xi).

### (i: Sample preparation step)

First, prepare two different states of Protein samples I and II. For example, Protein sample I may be a protein sample to be analyzed and Protein sample II may be a sample containing the control proteins for the protein contained in Protein sample I. More concretely, Protein sample I for analysis may be a protein sample of pathologic state, and control Protein sample II may be a protein sample of normal state. In the present invention, quantitative analysis of expressed proteomes between these Protein sample I and Protein sample II is performed. In the present invention, the protein samples may include molecules having a relatively low molecular weight such as peptides.

### (ii: Solubilization step)

This step is conducted individually on Protein sample I and Protein sample II under the same condition.

In this step, a protein sample is solubilized using urea or guanidine hydrochloride which is a denaturing agent. Concentration of the denaturing agent is not particularly limited, and may be appropriately determined by the one skilled in the art in consideration of the kind of the protein sample, other conditions or the like, so as to make the protein sample solubilize and denature. For example, urea may be used in an aqueous solution having a concentration of 2 M to a saturated concentration, preferably 2 M to 10 M. More preferably, urea is used in an aqueous solution of 8 M. Guanidine hydrochloride may be used in an aqueous solution having a concentration of 1.5 M to a saturated concentration, preferably 1.5 M to 8 M. More preferably, guanidine hydrochloride is used in an aqueous solution of 6 M. Moreover, the denaturing agent may be used in combination with EDTA of about 5 mM.

Further, the above denaturing agents may be used in such an amount that the denaturing agent is 5 to 50 µl per 100 µg of the protein sample. A preferred amount is about 25 µl. For example, when the protein sample is in a freeze-dried powder state, 25 µl of denaturing agent may be used, while when the protein sample is a solution state, the denaturing agent may be used with the concentration of the denaturing agent adjusted so that the final volume is 25 µl.

A temperature of solubilization may be 0 to 30°C, preferably at about room temperature when urea is used; and at a temperature of 0 to 100°C, preferably at about room temperature when guanidine hydrochloride is used.

### (iii: Modification step)

In this step, the protein samples solubilized in the manner as described above are modified using two modification reagents, namely isotope-labeled 2-nitro[¹³C₆]benzenesulfenyl chloride (NBSCl(heavy)) and isotope-unlabeled 2-nitro[¹²C₆]benzenesulfenyl chloride (NBSCl (light)). These modification reagents of the present invention selectively modify, namely label a tryptophan residue in a protein. Protein sample I is modified with either one of NBSCl(heavy) and NBSCl(light), and separately from this, Protein sample II is modified with the other one of NBSCl(heavy) and NBSCl(light).

The modification reagent is preferably used as a solution of acetic acid. For example, a solution dissolving 0.17 mg of 2-nitrobenzenesulfenyl chloride in 25 µl of acetic acid may be used. Such a modification reagent may be used in great excess amount of about 20 equivalents of the protein sample. For example, with respect to 100 µg of the protein sample, a modification reagent solution may be used so as to contain 0.17 mg of 2-nitrobenzenesulfenyl chloride. In this way, quite nearly 100% of tryptophan residues in the protein sample are modified.

Strictly speaking, 2-nitrobenzenesulfenyl chloride strictly has a difference in molecular mass of about 3% between the NBSCl(light) and the NBSCl(heavy). However, when mass of 2-nitrobenzenesulfenyl chloride is described herein, the difference in mass of these substances at the same molar amount is regarded as calculationally negligible. Therefore, the same mass may used for these two substances.

The modification reaction may be conducted by adding a modification reagent to protein samples, and incubation. About four hours is sufficient for allowing the reaction. In other words, the reaction may be ended right after starting of the reaction or within four hours. The standard protocol may employ a reaction time of one hour.

In this manner, the present invention significantly reduces the modification reaction time compared to the conventional NBS method. For this reason, it becomes possible to shorten the operation time required for executing the overall protocol of three days, in turn, to two days.

### (iv: Desalting step)

In this step, the modified Protein sample I and the modified Protein sample II obtained in the above step (iii) are mixed, and then the resultant protein sample mixture is subjected to desalting. As a desalting method, any conventional method may be used without any restriction. For example, desalting may be conducted using a Sephadex LH-20 column and an aqueous solution of acetonitrile.

### (v: Resolubilization step)

In this step, the desalted protein sample mixture obtained in the above step (iv) is resolubilized using urea or guanidine hydrochloride serving as a denaturing agent. The concentration of the denaturation agent is not particularly limited, and may be appropriately determined by one skilled in the art in consideration of the kind of the protein sample, other conditions or the like so that the solubilization and the denaturing of the protein sample will occur. For example, urea may be used as an aqueous solution having a concentration of 2 M to a saturated concentration, preferably 2 M to 10 M. More preferably, urea is used as an aqueous solution of 8 M. Guanidine hydrochloride may be used as an aqueous solution having a concentration of 1.5 M to a saturated concentration, preferably 1.5 M to 8 M. More preferably, guanidine hydrochloride is used as an aqueous solution of 6 M. In the present step, Tris HCl (pH approx. 8.8) of about 50 mM is added as a buffer for the purpose of pH adjustment in the subsequent reduction and alkylation step.

In the present invention, the conditions defined in the above solubilization step (ii) and the resolubilization step (v) can avoid the problem of aggregation that have often occurred in protein samples conventionally. Therefore, it is possible to keep the solubility until the later-described digestion step (vii) with least loss of sample. The later described Experimental Example C-1 and Experimental Example C-2 practically demonstrated such an effect.

As described above, since the method of the present invention can significantly reduce the sample loss compared to the conventional NBS method, the coverage of NBS modified peptide ion detected in mass spectrometry is dramatically improved. This effect enables the present invention to be usefully applied in analysis of, in particular, samples derived from living organism such as sera or extracts from organs. The later-described Experimental Example C-3 practically demonstrated such an effect.

Furthermore, the present invention significantly improves the quantitativeness compared to the conventional NBS method. The later described Experimental Example C-4 practically demonstrated such an effect.

### (vi: Reduction and Alkylation step)

In this step, conventional reagents and reaction conditions may be used without any restriction. Alkylation mainly occurs at a sulfhydryl group, however, alkylation may also occur at an imidazolyl group, an amino group or the like. Therefore, in the conventional NBS method, alkylation will occur not only at an amino acid residue having a sulfhydryl group such as cysteine, but also partially occur at an amino acid residue having other amino groups in some cases. As a result, a peak larger by *m*/*z* value of 57 than a peak of objective peptide (+57(m/z) peak) is sometimes observed in a mass spectrum. While in the present invention, a reaction solution of this step contains urea or guanidine hydrochloride. These substances have an amino group. Partial alkylation that has conventionally occurred at other amino acid residues having an imidazolyl group or an amino group would occur at an amino group of urea or guanidine hydrochloride which is present in the reaction system of the present invention. In other words, alkylation which is a side reaction occurred in the conventional method would be competitively inhibited. As a result, in the present invention, the aforementioned +57(m/z) peak is hardly detected in a mass spectrum. The later-described Experimental Example C-5 practically demonstrated such an effect.

### (vii: Digestion step)

In this step, trypsin digestion is conducted. This step is conducted in the presence of urea or guanidine hydrochloride which is a denaturing agent. The concentration of the denaturing agent may be 0.08 M to 4 M, preferably 0.8 M to 1.6 M in the case of urea, and 0.06 M to 3 M, preferably 0.6 M in the case of guanidine hydrochloride. In this step, CaCl₂ of approx. 5 mM is preferably added for the purpose of stabilization of trypsin structure and its activation.

In the present step, the denaturing agent remaining in the sample of the above resolubilization step is available. For example, a trypsin digestion buffer may be added to the sample after completion of the resolubilization step and the reduction and alkylation step so that a desired concentration is achieved. As other conditions for digestion, pH is adjusted so as to be around the optimum pH of trypsin enzyme, and reaction time may be about 4 to 16 hours at 37°C normally. In this manner, a peptide mixture containing modified peptide fragments and unmodified peptide fragments is obtained.

### (viii: Enrichment/Separation step)

In this step, modified peptides are selectively enriched from the above peptide mixture using a media having a phenyl group. This step utilizes the unique selectivity due to the π-π electrons interaction acting between π-electron compounds. To be more specific, the media exerts excellent retention ability to the modified peptide through the interaction between a π electron possessed by an indole group of tryptophan and a nitrophenylthio group in the modified peptide and a π electron possessed by a phenyl group on the media, allowing selective enrichment and separation. Such a media is appropriately selected from Hi-Trap phenyl FF, Hi-Trap phenyl HP, Phenyl Sepharose 6 Fast Flow, Phenyl Sepharose High Performance (there are available from Amersham Biosciences), YMC*GEL Ph (YMC Corporation) and the like, and used as a phenyl column.

On the other hand, in the conventional NBS method using a LH-20 column, a significant number of unmodified peptides were mingled in an eluted fractions. According to the present invention, the number of mingled unmodified peptides is decreased, and especially, unmodified peptides detected around m/z value of 1200-1700 that are observed a lot in the conventional NBS method are hardly observed. Additionally, in the conventional NBS method, each of peptides is eluted in almost all of the eluted fractions in the enrichment/separation step, while the present invention can separate the each of peptides each other to some extent. Accordingly, the present invention is superior in coverage of detected peptides to the conventional method. The later-described Experimental Example C-6 practically demonstrated such an effect.

### (ix: Mass spectrometry step)

The modified peptides enriched and separated in the above step (viii) are subjected to mass spectrometry. When a MALDI mass spectrometer is used in measurement of the present invention, a MAIDI-IT-TOF mass spectrometer (for example, AXIMA-QIT available from SHIMADZU) and the like may be used in addition to the MALDI-TOF mass spectrometer (for example, AXIMA-CFR, available from SHIMADZU) used in the conventional NBS method.

When a MALDI-TOF mass spectrometer is used, 4-CHCA (α-cyano-4-hydroxycinnamic acid) or the like is used as a matrix. On the other hand, when a MAIDI-IT-TOF mass spectrometer is used, 3-CHCA (α-cyano-3-hydroxycinnamic acid) or 3H4NBA (3-hydroxy-4-nitrobenzoic acid) is used as a matrix.

A person skilled in the art may appropriately determine the use form of these compounds in view of the use as a matrix for mass spectrometry. For example, these compounds are preferably used in a solution state. For example, the solution may be used at a concentration of 1 mg/ml to a saturated concentration.

Preferred solvents used in preparing the above solution include an aqueous solution of acetonitrile, an aqueous solution of trifluoroacetic acid (TFA), or an aqueous solution of acetonitrile-trifluoroacetic acid (TFA). When the aqueous solution of acetonitrile or the aqueous solution of acetonitrile-TFA is used, the concentration of acetonitrile may be, but not limited to, not more than 90%, preferably about 50%. When the aqueous solution of TFA or the aqueous solution of acetonitrile-TFA is used, the concentration of TFA may be, but not limited to, not more than 1%, preferably about 0.1%.

As for 4-CHCA, 4-CHCA is dissolved in the above solvent and may be used as a matrix solution having a concentration of 1 mg/ml to a saturated concentration, preferably 10 mg/ml.

As for 3-CHCA, 3-CHCA is dissolved in the above solvent and may be used as a matrix solution having a concentration of 1 mg/ml to a saturated concentration, preferably 10 mg/ml.

Further, as for 3H4NBA, 3H4NBA is dissolved in the above solvent and used as a matrix solution having a concentration of 1 mg/ml to a saturated concentration, preferably a saturated concentration.

The amount expressed as % in this description is on the basis of v/v% unless otherwise specified.

In identifying a sequence of a peptide existing at different abundances in different samples, MS/MS analysis using a mass spectrometer equipped with a quadrupole ion trap (QIT) such as an AXIMA-QIT (available from SHIMADZU) is more favorable than PSD analysis by an AXIMA-CFR (available from SHIMADZU) from the view point of sensitivity. In measurement using such an ion trap type mass spectrometer, DHB (2,5-dihydroxy benzoic acid) is usually used as a matrix. However, when DHB is used as a matrix, NBS-modified peptides are hardly detected. Accordingly, 3-CHCA or 3H4NBA is used as a matrix when measurement is conducted using an ion trap type mass spectrometer in the present invention. This enables efficient ionization of NBS-modified peptides. Therefore, the efficiency of MS/MS analysis of NBS-modified peptides in the present invention is dramatically improved in comparison with the conventional NBS method. The later-described Experimental Example C-7 practically demonstrated such an effect.

In the present invention, when 3-hydroxy-4-nitrobenzoic acid (3H4NBA) is used as a matrix, 3H4NBA is preferably used as a mixed matrix in which 3H4NBA is combined with α-cyano-4-hydroxycinnamic acid (4-CHCA). (Hereinafter, a singularly used matrix in which the matrix is used without combining with 4-CHCA, and a mixed matrix in which 4-CHCA is used in combination are sometimes simply described as matrix.)

3H4NBA and 4-CHCA may be combined, for example, in the following quantitative relationship in a nonrestrictive manner.

3H4NBA may be prepared at a concentration as described above. Specifically, a 3H4NBA solution may be prepared as an aqueous solution having a concentration of 1 mg/ml to a saturated concentration; for example, when an aqueous solution of acetonitrile, an aqueous solution of TFA, or an aqueous solution of acetonitrile-TFA is used as a solvent, a 3H4NBA solution may be prepared as a solution having a concentration of 1 mg/ml to a saturated concentration, preferably a saturated concentration.

On the other hand, 4-CHCA may also be prepared at a concentration as described above. Specifically, a 4-CHCA solution may be prepared as an aqueous solution having a concentration of 1 mg/ml to a saturated concentration; for example, when an aqueous solution of acetonitrile, an aqueous solution of TFA, or an aqueous solution of acetonitrile-TFA is used as a solvent, 4-CHCA solution may be prepared as a solution having a concentration of 1 mg/ml to a saturated concentration, preferably 10 mg/ml.

The both of solution prepared in these manners are mixed in a volume ratio of, preferably 1:10 to 10:1, more preferably 1:3 to 3:1, for example 1:1 for use.

The conventional matrix 4-CHCA has a drawback that self-disintegration of an analyte to be measured occurs during measurement with a MALDI spectrometer such as MALDI-IT, MALDI-IT-TOF, or MALDI-FTICR spectrometer in which a time from ionization to detection of ion is long. However, the conventional matrix 4-CHCA shows excellent measuring sensitivity and is advantageous in that an optimum spot on which a laser is to be focused can be easily found in a mass spectrometric sample.

On the other hand, the matrix 3H4NBA of the present invention can advantageously suppress the progression of self-disintegration of an analyte to be measured, and achieve specific ionization of a hydrophobic analyte, especially of a NBS-modified peptide. The matrix 3H4NBA of the present invention is used in combination with 4-CHCA, thereby synergistic effect of the advantages given by both of the matrices is exerted. In brief, an ability to detect with high sensitivity possessed by 4-CHCA is added while maintaining an ability to detect with specificity possessed by 3H4NBA by itself, and achievability of ionization of hydrophobic sample, and it is possible to conduct a mass spectrometry with higher analytical efficiency. The later-described Experimental Examples C-8 and C-9 practically demonstrated such an effect.

A concrete protocol of the present invention will be described below. This protocol is described for treating each 100 µg of the samples in states I and II. (NBSC1 and NBS-modified peptides should be kept from light as much as possible during this protocol.) As is already mentioned, any amount expressed by % in this protocol and later-described Experimental Examples is based on v/v % unless otherwise specified.

### [Solubilization of sample (separately executed for Samples of state I and state II)]

1. Prepare each 100 µg of samples of "state I" and "state II".
2. Lyophilize each sample (or dry to solid using a vacuum concentrator).
3. Dissolve each sample in 25 µl of a denaturing buffer (8 M aqueous solution of urea containing 5 mM EDTA, or 6 M aqueous solution of guanidine hydrochloride containing 5 mM EDTA).
4. Stir well on a Vortex mixer.

### [Modification of tryptophan residue with NBSCl

### (separately executed for Samples of state I and state II)]

1. Add 25 µl of NBS reagent (light) solution (acetic acid solution containing 0.17 mg of NBSCl (light)) to the sample of "state I", and stir on a Vortex mixer, separately add 25 µl of NBS reagent (heavy) solution (acetic acid solution containing 0.17 mg of NBSCl (heavy)) to the sample of "state II", and stir on a Vortex mixer.
2. Incubate each sample for one hour under gentle stirring.

### [Desalting of reaction solution and removal of excess NBS reagent (the two modified samples are mixed in this step)]

1. Prepare an LH-20 column (equilibrate 500 µl of LH-20 in advance with 30% acetonitrile aqueous solution), and allow the supernatant to freely fall to the level of the resin layer.
2. Gently apply a mixed sample (mixture of NBSC1(light)-modified sample and NBSCl(heavy)-modified sample, total volume 100 µl) on the column. (Discard flow-through fractions.)
3. Wash the column with 100 µl of 30% acetonitrile aqueous solution.
4. Elute the desalted sample with 200 µl of 30% acetonitrile aqueous solution.
5. Lyophilize the eluted fractions.

### [Reduction and alkylation]

1. Dissolve the sample in 48 µl of 8 M aqueous solution of urea containing 50 mM Tris HCl (pH8.8) or 6 M aqueous solution guanidine hydrochloride containing 50 mM Tris HCl (pH8.8).
2. Add 1 µl of a reduction solution (200 mM TCEP aqueous solution) and gently stir.
3. Incubate for 30 min. at 37°C.
4. Add 1 µl of an alkylation solution (500 mM iodoacetamide aqueous solution) and gently stir.
5. Incubate at room temperature for 45 min.

### [Trypsin digestion]

1. Dissolve 10 µg of trypsin (Promega, sequencing grade) in 450 µl of a digestion buffer (50 mM Tris HCl (pH7.8), 5 mM CaCl₂).
2. Add the trypsin solution to the sample and gently mix by pipetting.
3. Incubate at 37°C for 4 to 16 hours.
4. Add 50 µl of 1% TFA aqueous solution (final concentration 0.1%) before loading on the column. Hydrochloric acid may be added in place of TFA. In such a case, the hydrochloric acid may be adjusted so that its final concentration is 10 mM.

### [Enrichment of modified peptides]

1. Fill an open column with 1 ml of phenyl column such as Phenyl Sepharose^{™} High Performance (Amersham Biosciences).
2. Equilibrate the column with 5 ml of water, followed by 5 ml of 0.1% TFA aqueous solution.
3. Apply 550 µl of digested sample on the column (collect this eluate as "Flow-through" fraction).
4. Wash the column with 1 ml of 0.1% TFA aqueous solution (collect this eluate as "Wash" fraction).
   Repeat this step twice more (three times in total).
5. Elute with 0.5 ml of an elution buffer (0.1% TFA aqueous solution containing 10% acetonitrile) (collect this eluate as "Elute" fractions), and repeat this step once more.
6. Repeat the step 5. until the concentration of acetonitrile is 40% while increasing the concentration of acetonitrile by 5% at a time.
7. (Optional step: conduct as necessary). For MS analysis, desalt and enrich peptides from the "Flow-through" fraction and "Wash" fractions using ZipTip.
8. Dry the "Elute" fractions to solid by means of a vacuum concentrator. For MS analysis or further separation using HPLC or the like, suspend the dried solid sample in about 5 to 50 µl of 0.1% TFA aqueous solution.

Hydrochloric acid may be used in place of the 0.1% TFA aqueous solution used in enrichment of the modified peptides. In this case, the hydrochloric acid may be adjusted so that its concentration is 10 mM.

### [Mass spectrometry]

The sample may be directly subjected to MS analysis, or may be further fractionated using HPLC and the like. When MALDI-MS measurement is conducted, the sample is mixed with a matrix solution for conducting MS measurement.

The present invention further provides a kit containing 2-nitro[¹³C₆]benzenesulfenyl chloride, 2-nitro[¹²C₆]benzenesulfenyl chloride, and a media having a phenyl group. These 2-nitro [¹³C₆]benzenesulfenyl chloride and 2-nitro[¹²C₆]benzenesulfenyl chloride may be used, for example, as modification reagents for conducting the above modification step (iii). The media having a phenyl group may be used for conducting the above enrichment/separation step (viii).

Therefore, the kit of the present invention may be used for conducting a global quantitative analysis of protein, for example, the protocol described above.

More specifically, the kit of the present invention contains 2-nitro[¹³C₆]benzenesulfenyl chloride (NBSCl(heavy); NBS(heavy) reagent) and 2-nitro[¹²C₆]benzenesulfenyl chloride (NBSCl(light); NBS(light)reagent) serving as modification reagents for conducting the above modification step (iii) and a media having a phenyl group for conducting the above enrichment/separation step (viii), and preferably includes a denaturing agent and/or a matrix. The kit of the present invention may contain various solvents used in the method of the present invention as described above.

The denaturing agent may be used for conducting the above solubilization step (ii) and the above resolubilization step (v). Therefore, as the denaturing agent, urea or guanidine hydrochloride as described in the solubilization step (ii) and the resolubilization step (v) is preferably used. The denaturing agent may be dissolved in solvents described in the solubilization step (ii) and the resolubilization step (v). For example, when guanidine hydrochloride is employed as a denaturing agent, it may be provided as it is dissolved in a solvent. In such a case, guanidine hydrochloride may be an aqueous solution having a concentration of 1.5 M to saturated concentration, preferably 1.5 M to 8 M, more preferably 6 M. When urea is dissolved in a solvent as a denaturing agent, may be an aqueous solution of urea having a concentration of 2 M to saturated concentration, preferably 2 M to 10 M, more preferably 8 M.

The matrix may be used for conducting the above mass spectrometry step (ix). Therefore, the matrix is preferably selected from 4-CHCA (α-cyano-4-hydroxycinnamic acid), 3-CHCA (α-cyano-3-hydroxycinnamic acid), and 3H4NBA (3-hydroxy-4-nitrobenzoic acid) as described in the mass spectrometry step (ix). Also a set of 3H4NBA and 4-CHCA is preferred for use as the mixed matrix.

These matrices and auxiliary matrices may be dissolved in solvents as described in the mass spectrometry step (ix). For example, the matrix may be as a solution having a concentration of 1 mg/ ml to saturated concentration. When an acetonitrile aqueous solution, TFA aqueous solution, or an acetonitrile-TFA aqueous solution is used as a solvent, α-cyano-3-hydroxycinnamic acid may be as a solution having a concentration of 1 mg/ml to saturated concentration, preferably 10 mg/ml; 3-hydroxy-4-nitrobenzoic acid may be as a solution having a concentration of 1 mg/ml to saturated concentration, preferably saturated concentration; and α-cyano-4-hydroxycinnamic acid may be as a solution having a concentration of 1 mg/ml to saturated concentration, preferably 10 mg/ml. Also, a mixed solution in which a solution of 3-hydroxy-4-nitrobenzoic acid and a solution of α-cyano-4-hydroxycinnamic acid, among these solutions, in a volume ratio of preferably 1:10 to 10:1, more preferably 1:3 to 3:1, for example 1:1 is exemplified.

The kit of the present invention may further contain a desalting column and filling gel for the desalting column; a reduction reagent and an alkylation reagent; trypsin; and a column for filling the media having a phenyl group. The desalting column and filling gel for the desalting column may be used for conducting the above desalting step (iv), for example. The reduction reagent and the alkylation reagent may be used for conducting the above reduction and alkylation step (vi), for example. Trypsin may be used for conducting the above digestion step (vii), for example. The column for filling the media having a phenyl group may be used for conducting the enrichment/separation step (viii), for example. In other words, the column for filling the media having a phenyl group may be used as an enrichment column, and the media having a phenyl group may be used as filling gel for the enrichment column.

The kit of the present invention enables to conduct a global quantitative analysis of protein of the present invention as described above, and therefore, brings the following effect as already described in the method of the present invention.
· Decrease in sample loss
· Reduction of +57(m/z) band
· Reduction in rate of mingled unmodified peptides
· Improvement in separating efficiency of each peptide
· Realization of MS/MS analysis using QIT
· Increase in number of detectable pair of peaks
· Shortening in total operation time for the protocol
· Improvement in quantitiativeness

### EXAMPLES

The first invention will now be explained more detail by way of examples A-1 and A-2, however, the present invention is not limited to these examples. As is mentioned above, the amount expressed as % in this description is on the basis of v/v% unless otherwise specified. In Examples, labeling with an NBS reagent is referred to as modification or NBS modification in some cases.

### [Example A-1]

In this Example, measurement was conducted by means of a mass spectrometer using NBS modified peptides (a mixture of peptides modified with an NBS (heavy) reagent labeled with 6 stable isotope elements ¹³C and peptides modified with an unlabeled NBS (light) reagent) as a sample to be measured, using a matrix of 3-CHCA (α-cyano-3-hydroxycinnamic acid, Formula A-I) and 3H4NBA (3-hydroxy-4-nitrobenzoic acid, Formula A-II) of the present invention and using a conventional matrix DHB (2,5-dihydroxybenzoic acid, Formula A-IV) for comparison.

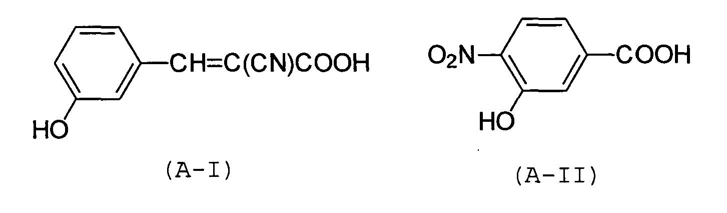

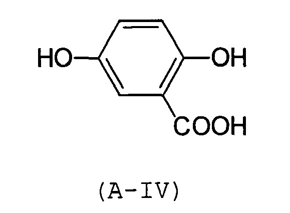

The sample to be measured was prepared in the following manner.

Two sample mixtures each having a total weight of 100 µg given by each 25 µg of four purified proteins (ovalbumin, glyceraldehyde-3-phosphate dehydrogenase, lysozyme, and α-lactalbumin, all available from SIGMA) was mixed were prepared. Samples to be measured were prepared in accordance with a protocol for "¹³CNBS Isotope Labeling Kit" (SHIMADZU) except that solubilization for each mixture and resolubilization for NBS-modified sample mixture were conducted using urea having a final concentration of 8M as a denaturing agent. One sample mixture of the two sample mixtures was modified with a NBS Reagent (heavy) (2-nitro [¹³C₆] benzenesulfenyl chloride) and the other sample mixture was modified with a NBS Reagent (light) (2-nitro [¹²C₆] benzenesulfenyl chloride). Mixing of the both of the modified samples, desalting, resolubilization by urea, reduction, alkylation, and trypsin digestion were conducted.

Thereafter, the samples were lyophilized, suspended in 500 µl of 0.1% trifluoroacetic acid (TFA) aqueous solution, and loaded on a HiTrap Phenyl FF (high sub) column (Amersham Bioscience) equilibrated with 0.1 % TFA aqueous solution. After washing this column with 3 ml of 0.1% TFA aqueous solution, 1 ml of 0.1% TFA aqueous solution containing 10% acetonitrile was used for elution. Then elution was similarly continued while varying the concentration of acetonitrile of TFA aqueous solution for elution from 15, 20, 25, 30, 35, and 40% in this order. Following the elution, the fraction eluted with 0.1% TFA aqueous solution containing 10% acetonitrile was lyophilized, resuspended in 50 µl of 0.1% TFA aqueous solution, and desalted with ZipTip (µC18). Thus a sample to be measured was obtained.

As the matrix, three kinds including 3-CHCA (α-cyano-3-hydroxycinnamic acid, Formula A-I) and 3H4NBA (3-hydroxy-4-nitrobenzoic acid, Formula A-II) of the present invention, and a conventional matrix DHB (2,5-hydroxybenzoic acid, Formula A-IV) for comparison were used.

The matrix for use was prepared in the following manner. Using 50% acetonitrile aqueous solution containing 0.1% TFA as a solvent, each of 3-CHCA, 3H4NBA and DHB was dissolved. DHB and 3-CHCA were prepared into respective solutions of 10 mg/ml, and 3H4NBA was prepared into a saturation solution.

Each 0.5 µl of the solution of a sample to be measured and the matrix solution obtained as described above was taken and mixed, and dropped on a target plate. After drying the solution, measurement was conducted using an AXIMA-QIT apparatus (MALDI-IT-TOF mass spectrometer, SHIMADZU).

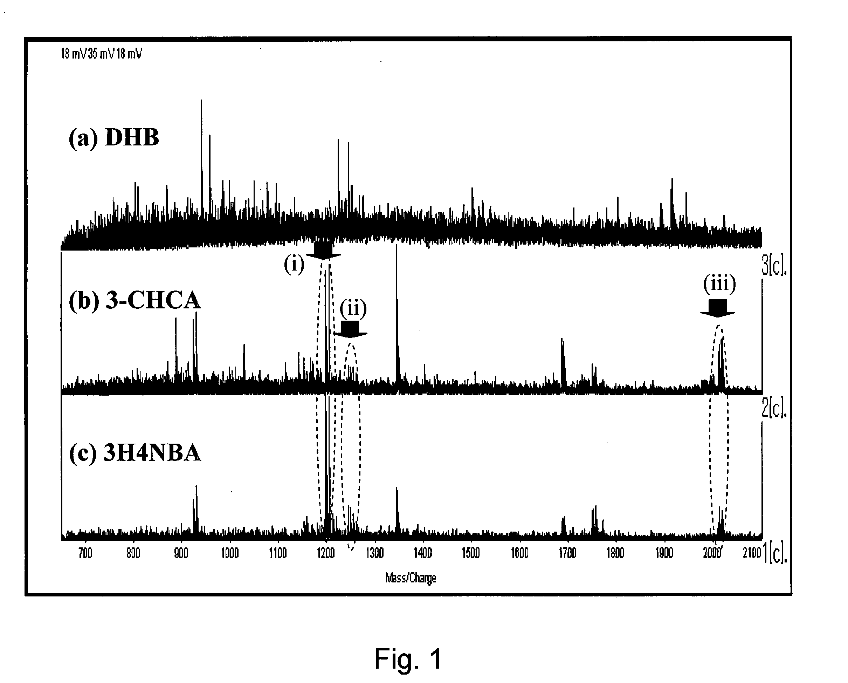

A MS spectrum obtained by the AXIMA-QIT apparatus is shown in Fig.1. In Fig. 1, the horizontal axis represents mass-to-charge ratio, and the vertical axis represents relative intensity of ion. (a) is a spectrum of NBS-modified peptides when DHB was used as the matrix; (b) is a spectrum of NBS-modified peptides when 3-CHCA was used as the matrix; and (c) is a spectrum of NBS-modified peptides when 3H4NBA was used as the matrix. In the figure, (i), (ii), and (iii) indicate pair of peaks of NBS-modified peptide. Each pair of peaks were detected as a pair of peaks having a difference in m/z value of 6 that is corresponding to a difference in mass between the two modification reagents, that is, between the NBS Reagent (heavy) (2-nitro [¹³C₆] benzenesulfenyl chloride) and the NBS Reagent (light) (2-nitro[¹²C₆] benzenesulfenyl chloride). Theoretical mass values and sequence corresponding to each pair of peaks and protein from which each pair of peaks are derived are as follows.
(i) 1198.53, 1204.55 (m/z), GTDVQAWIR (SEQ ID NO: 1), lysozyme.
(ii) 1244.51, 1250.53 (m/z), LDQWLCEK (SEQ ID NO: 2), α-lactalbumin.
(iii) 2011.95, 2017.97 (m/z), ELINSWVESQTNGIIR (SEQ ID NO: 3), ovalbumin

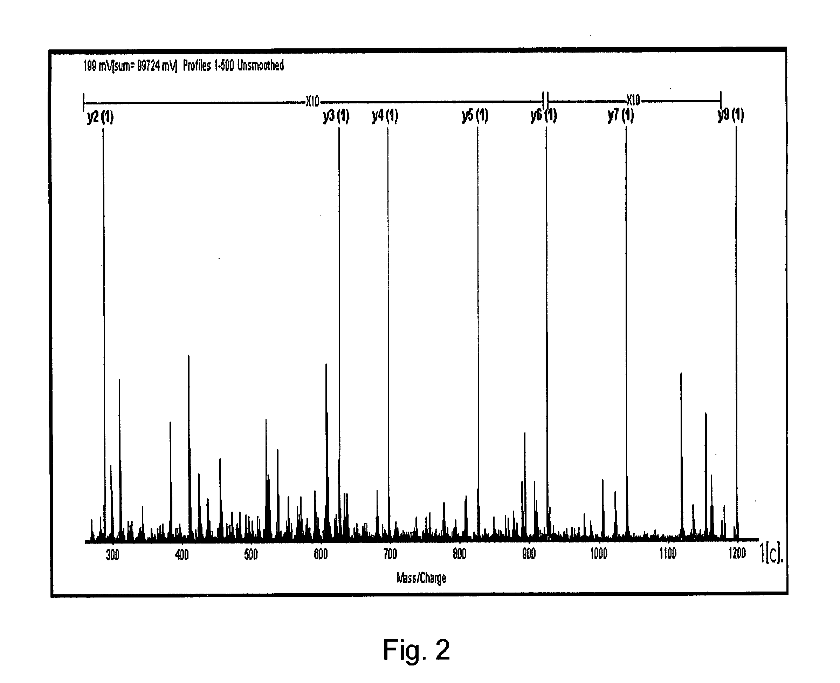

Fig. 2 is a fragment spectrum in a MS/MS analysis after selectively trapping the ion (GTDVQAWIR) corresponding to one of the paired peaks at m/z 1198.53 (i) in Fig. 1. In Fig. 2, the horizontal axis represents mass-to-charge ratio, and the vertical axis represents relative intensity of ion.

### [Example A-2]

### (a) Mass spectrometric measurement using a mass spectrometer with an ion trap and a mass spectrometer without an ion trap

Measurement was conducted by means of a mass spectrometers using a mixture of peptides modified with NBS reagent and unmodified peptides as a sample to be measured, and using a mixed matrix according to the present invention, namely a mixture of 3H4NBA (3-hydroxy-4-nitrobenzoic acid, Formula A-II) and conventional matrix of 4-CHCA (α-cyano-4-hydroxycinnamic acid, Formula A-III).

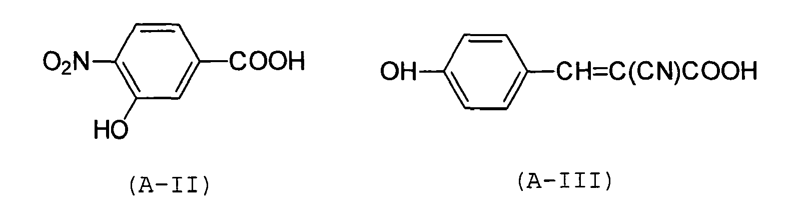

The sample to be measured was prepared in the following manner.

Two sample mixtures each having a total weight of 100 µg given by each 25 µg of four purified proteins (ovalbumin, glyceraldehyde-3-phosphate dehydrogenase, lysozyme, and α-lactalbumin, all available from SIGMA) was mixed were prepared. The protocol for "¹³CNBS Isotope Labeling Kit" (SHIMADZU) was followed except that each mixture was denatured using urea having a final concentration of 8M as a denaturing agent. Specifically, One of the two sample mixture was labeled-modified with a NBS Reagent (heavy) (2-nitro [¹³C₆] benzenesulfenyl chloride), and the other sample mixture was nonlabeled-modified with a NBS Reagent (light) (2-nitro[¹²C₆] benzenesulfenyl chloride). Mixing of the both of the modified samples, desalting, resolubilization by urea, reduction, alkylation, and trypsin digestion were conducted. The samples after digestion were conducted desalting treatment with ZipTip µ-C18, and eluting with 4 µl of 50% acetonitrile aqueous solution containing 0.1% TFA, to obtain a sample to be measured. 0.5 µl from this sample was applied on a target plate.

The matrix for use was prepared in the following manner. Each of 3H4NBA and 4-CHCA was dissolved in a solvent of 50% acetonitrile aqueous solution containing 0.1% TFA. 3H4NBA was prepared into a saturation solution, and 4-CHCA was prepared into a solution of 10 mg/ml. These solutions thus prepared were mixed with each other in a volume ratio of 1:1 to obtain a mixed matrix solution. On a prepared target plate on which a sample to be measured was applied, 0.5 µL of the mixed matrix solution was added. After drying, measurement was conducted using a MALDI-IT-TOF mass spectrometer having an ion trap (AXIMA-QIT, SHIMADZU) and a MALDI-TOF mass spectrometer without an ion trap (AXIMA-CFR plus, SHIMADZU).

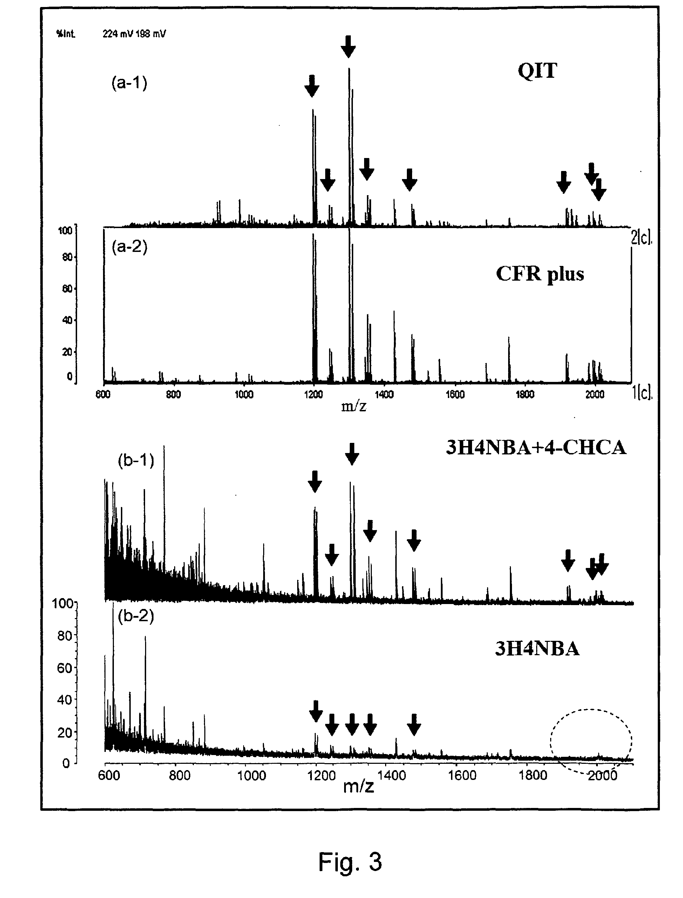

The MS spectra obtained in these measurements are shown in Fig. 3(a-1) and Fig. 3(a-2). In these figures, the horizontal axis represents mass-to-charge ratio (m/z), and the vertical axis represents relative intensity of ion (%int.). (a-1) is a spectrum obtained by using the AXIMA-QIT having an ion trap, and (a-2) is a spectrum obtained by using the AXIMA-CFR plus without an ion trap. Further, these figures show that the pairs of peaks marked with the arrows come from NBS-modified peptides. Each pair of peaks has a difference of m/z value of 6 that is corresponding to a difference in mass between the two modification reagents, that is, between the NBS Reagent (heavy) (2-nitro[¹³C₆] benzenesulfenyl chloride) and the NBS Reagent (light) (2-nitro[¹²C₆] benzenesulfenyl chloride).

As can be seen by comparison of the spectra of Fig. 3(a-1) and Fig. 3(a-2), almost the same spectrum was obtained. The fact that almost the same spectrum was obtained by a mass spectrometer having an ion trap and by a mass spectrometer without an ion trap indicates that self-disintegration of the analyte is suppressed in measurement using a mass spectrometer having an ion trap. This leads the conclusion that the matrix mixture of the present invention suppresses self-disintegration of an analyte to be measured that occurs during conventional measurement using a mass spectrometer with an ion trap (namely, mass spectrometer requiring relatively long time from ionization to detection of ion) using 4-CHCA alone as a matrix.

### (b) Mass spectrometry with a matrix of 3H4NBA by itself and a mixed matrix of 3H4NBA and 4-CHCA

Using the same sample to be measured as the above (a), a mass spectrometry was conducted with a matrix of 3H4NBA by itself and a mixed matrix of 3H4NBA and 4-CHCA which are the matrices of the present invention.

The same sample to be measured as used in the above (a) was diluted in 0.1% TFA aqueous solution to make a 1000-fold dilution, and 0.5 µl from the diluted solution was applied on a target plate.

As the matrix, a matrix of 3H4NBA by itself and a mixed matrix of 3H4NBA and 4-CHCA were used.

The matrix of 3H4NBA by itself was prepared as a saturated solution by dissolving 3H4NBA in 50% acetonitrile aqueous solution containing 0.1% TFA.

The mixed matrix of 3H4NBA and 4-CHCA was prepared in the same manner as in the above (a).

On a prepared target plate on which a sample to be measured was applied, the 3H4NBA solution or the mixed solution of 3H4NBA and 4-CHCA was applied. After drying, measurement was conducted using a MALDI-TOF (AXIMA-CFR plus, SHIMADZU).

The MS spectra obtained in these measurements are shown in Fig. 3(b-1) and Fig. 3(b-2). In these figures, the horizontal axis represents mass-to-charge ratio (m/z), and the vertical axis represents relative intensity of ion (%). (b-1) is a spectrum obtained by using the mixed matrix of 3H4NBA and 4-CHCA, and (b-2) is a spectrum obtained by using the 3H4NBA matrix. Further, these figures show that the pairs of peaks marked with the arrows come from NBS-modified peptides. Each pair of peaks has a difference of m/z value of 6 that corresponds to a difference in mass between the two modification reagents, that is, between the NBS Reagent (heavy) (2-nitro[¹³C₆] benzenesulfenyl chloride) and a NBS Reagent (light) (2-nitro[¹²C₆] benzenesulfenyl chloride).

As is apparent from comparison of spectra Fig. 3(b-1) and (b-2), the pairs of peaks of NBS-modified peptides are detected more sensitively in (b-1) than (b-2). This indicates that sensitivity is improved when as a matrix 3H4NBA is mixed with 4-CHCA. It was confirmed that advantages of 4-CHCA that "measurement with high sensitivity can realize" in the condition that "optimum spot on which a laser beam is to be focused can be readily found" are added while keeping the advantage of 3H4NBA that "hydrophobic sample can be detected by mass spectrometry." In brief, it was confirmed that the advantage of 3H4NBA that "self-disintegration of sample can be suppressed even if measurement is conducted using an ion trap MALDI mass spectrometer in which the time from ionization to detection of ion is relatively long" and the advantage of 4-CHCA "measurement with high sensitivity can realize" in the condition that "optimum spot on which a laser beam is to be focused can be readily found" were achieved simultaneously.

The above-described Examples A-1 and A-2 show concrete two modes within the scope of the present invention, however, the present invention can be carried out in various other modes. Therefore, the above-described Examples are merely illustrative in all respects, and must not be construed as being restrictive. Further, the changes that fall within the equivalents of the claims are all within the scope of the present invention.

The second invention will now be explained more detail by way of examples B-1 to B-8, however, the present invention is not limited to these examples. As is mentioned above, the amount expressed as % in this description is on the basis of v/v% unless otherwise specified. In Examples, labeling with a NBS reagent is also referred to as modification or NBS modification.

### [Example B-1]

In this Example, a mixture of ACTH(5-10) peptide (purchased from BACHEM) and ACTH(5-10) peptide modified with 2-nitro[¹²C₆] benzenesulfenyl chloride (NBS Reagent (light): SHIMADZU Corporation) was used as a sample to be measured, and measurement was conducted on a mass spectrometer using matrices of the present invention 3H4NBA (3-hydroxy-4-nitrobenzoic acid) and 4H3NBA (4-hydroxy-3-nitrobenzoic acid), and comparative conventional matrices DHB (2,5-dihydroxy benzoic acid) and 4-CHCA (α-cyano-4-hydroxy cinnamic acid).

A method for preparing samples to be measured will be described below.

Labeling of peptide was conducted by reacting 10 µg of ACTH for one hour in a sample solution prepared by adding 20 equivalents of NBS Reagent (light) in 50 µl of 70% acetic acid aqueous solution. After reaction, desalting using ZipTip (µC18) was conducted to give a modified sample. On the other hand, 10 µg of ACTH was subjected to stirring treatment for one hour in 50 µl of 70% acetic acid aqueous solution not containing NBS Reagent (light), and the resultant solution was desalted in the same manner as described above, to give an unmodified sample. The modified sample and the unmodified sample were mixed in equivalent amounts, to prepare a sample for mass spectrometry.

Four matrices including DHB, 4-CHCA, and two isomers of HNBA namely 3H4NBA and 4H3NBA, were prepared. These matrices were prepared by dissolving in 50% acetonitrile aqueous solution containing 0.1% TFA, and 4-CHCA, DHB and 4H3NBA were used as a 10 mg/ml solution, and 3H4NBA was used as a saturated solution.

0.5 µl of the above sample to be measured was taken and dropped on a target plate and dried, and then 0.5 µl of matrix solution was taken and dropped on the sample dried in advance and dried (the same operation was conducted in all following Examples). This operation was conducted for four matrices. Using target plate thus obtained, measurement was conducted using an AXIM-CFR apparatus (SHIMADZU Corporation).

The obtained spectra are shown in Fig. 4. In Fig. 4, the horizontal axis represents mass-to-charge ratio, and the vertical axis represents relative intensity of ion (hereinafter, in any figures, the horizontal axis represents mass-to-charge ratio, and the vertical axis represents relative intensity of ion). (a) is a spectrum when DHB was used as a matrix; (b) is a spectrum when 4-CHCA was used as a matrix; (c) is a spectrum when 3H4NBA was used as a matrix; and (d) is a spectrum when 4H3NBA was used as a matrix. The bold arrow represents a peak position of NBS-modified ACTH peptide, namely an objective peptide, and thin arrow represents a peak position of unmodified ACTH peptide. As shown in Fig. 4, in (c) and (d) using matrices of the present invention, an objective modified peptide was selectively detected.

### [Example B-2]

In this Example, measurement was conducted in a mass spectrometer by using a mixture of a labeled-modified protein and an unlabeled-modified protein as a sample to be measured, and using a matrix 3H4NBA of the present invention and a comparative conventional matrix 4-CHCA.

Two sample mixtures each having a total weight of 100 µg given by each 25 µg of four purified proteins (ovalbumin, glyceraldehyde-3-phosphate dehydrogenase, lysozyme, and α-lactalbumin, all available from SIGMA) was mixed were prepared. According to a protocol "¹³CNBS Isotope Labeling Kit" (SHIMADZU), one sample mixture was modified with 2-nitro[¹³C₆] benzenesulfenyl chloride (NBS Reagent (heavy); SHIMADZU) and the other sample mixture was modified with 2-nitro[¹²C₆] benzenesulfenyl chloride (NBS Reagent (light); SHIMADZU). These two modified samples were subjected to mixing and desalting followed by reduction, alkylation and trypsin digestion. The samples were lyophilized, resuspended in 50 µl of 0.1% TFA aqueous solution, and subjected to desalting treatment with ZipTip(µC18). Using the resultant solution as a sample to be measured, and using the same matrix solutions of 4-CHCA and 3H4NBA as in Example B-1, measurement was conducted in the same manner as in Example B-1 by a mass spectrometer.

The obtained spectra are shown in Fig. 5. In Fig. 5, (a) is a spectrum when 4-CHCA was used as a matrix and (b) is a spectrum when 3H4NBA was used as a matrix. In Fig. 5, among the peaks detected in (a), pairs of peaks of the objective modified peptides having a difference of *m*/*z* value of 6 or 12 that is corresponding to a mass difference between the two reagents used in the modification, the NBS Reagent (heavy) (2-nitro[¹³C₆] benzenesulfenyl chloride) and the NBS Reagent (light) (2-nitro[¹²C₆] benzenesulfenyl chloride) or a mass difference between their multiples were selectively detected in (b) using a matrix of the present invention.

### [Example B-3]

In this Example, using a mixture of DSIP peptide (delta sleep-inducing peptide (Peptide Institute): 500 fmol) and DSIP peptide modified with NBS Reagent (light) (500 fmol) as a sample to be measured, and using the matrix 3H4NBA of the present invention and the comparative conventional matrix 4-CHCA, measurement was conducted using a mass spectrometer.

Modification was conducted in the same manner as in Example B-1 except that DSIP peptide was used as a sample for modification, to obtain a sample to be measured. Using the same matrix solutions of 4-CHCA and 3H4NBA as in Example B-1 as matrices, measurement was conducted using a mass spectrometer in the same manner as in Example B-1. The obtained spectrua are shown in Fig. 6. In Fig. 6, (a) is a spectrum using 4-CHCA as a matrix, and (b) is a spectrum using 3H4NBA as a matrix. The bold arrow represents a position of NBS modified DSIP peptide, and the part surrounded by the ellipse of dotted line shows the peaks smaller than the peak indicated by the arrow by m/z value of 16, 32, and 155, respectively. These peaks observed in (a) were hardly observed in (b) using the matrix of the present invention. This demonstrates that the peaks of the objective peptides were selectively detected.

### [Example B-4]

In this Example, lysozyme was reduced and alkylated, and then, nitrated and digested with trypsin. Measurement was conducted by a mass spectrometer using the resultant digested product as a sample to be measured, and using the matrix 3H4NBA of the present invention and the comparative conventional matrix 4-CHCA.

Now, a preparation method of sample to be measured will be described.

First, 1 mg of lysozyme was reduced and alkylated according to a conventional method. Next, according to the method described in Riordan J.F. et al., 1996; Sokolovsky M. et al., 1966, nitration was conducted using tetranitro methane. Thereafter, trypsin was added according to a conventional method to digest into peptide fragments. A portion of the obtained digested product corresponding to 14 µg was lyophilized and then dissolved in 50 µl of 0.1% TFA aqueous solution, and desalted with ZipTip µ-C18. Thus obtained digested product was used as a sample to be measured. In this sample to be measured, tyrosine residues of a peptide fragment was partly nitrated. That is, this sample was a mixture of peptide fragment containing nitrotyrosine, peptide fragment containing tyrosine not nitrated, and peptide fragment not containing tyrosine.

As a matrix, the comparative conventional 4-CHCA (α-cyano-4-hydroxycinnamic acid: purchased from SIGMA), or 3H4NBA (3-hydroxy-4-nitrobenzoic acid: purchased from ALDRICH) was used. These matrices were dissolved in 50% acetonitrile aqueous solution containing 0.1% TFA and 4-CHCA was used as a solution of 10 mg/ml and 3H4NBA was used as a saturated solution.

Using the above matrix, the above sample to be measured was subjected to MS measurement in a reflectron mode using a mass spectrometer AXIMA-CFR (SHIMADZU). In every following Example, MS measurement was also conducted in a reflectron mode using the AXIMA-CFR. The obtained spectra are shown in Fig. 7 and Fig. 8 (A and B). Fig. 8A is an enlarged view of m/z value of 850 to 960 part in Fig. 7, and Fig. 8B is an enlarged view of *m*/*z* value of 1740 to 1820 part of Fig. 7. In Fig. 7 and Fig. 8 (A and B), (a) is a spectrum measured using 4-CHCA as a matrix and (b) is a spectrum measured using 3H4NBA as a matrix.

The peaks indicated by the filled arrows ((2*): m/z=1798.83; Asn-Thr-Asp-Gly-Ser-Thr-Asp-Tyr*-Gly-Ile-Leu-Gln-Ile-Asn-Ser-Arg (SEQ ID NO: 4) and (8*): m/z=919.41; His-Gly-Leu-Asp-Asn-Tyr*-Arg (SEQ ID NO: 5), wherein Tyr* represents a nitrated tyrosine residue) are peaks of objective trypsin-digested peptides containing nitrated tyrosine residue.

The peaks indicated by the open arrows ((2): m/z=1753.84; Asn-Thr-Asp-Gly-Ser-Thr-Asp-Tyr-Gly-Ile-Leu-Gln-Ile-Asn-Ser-Arg (SEQ ID NO: 6) and (8): m/z=874.42; His-Gly-Leu-Asp-Asn-Tyr-Arg (SEQ ID NO: 7)) are peaks of digested peptides having the same sequences as the above objective digested peptides except that they failed to be nitrated.

The peaks not indicated by arrows (3): m/z=1675.80; Ile-Val-Ser-Asp-Gly-Asn-Gly-Met-Asn-Ala-Trp-Val-Ala-Trp-Arg (SEQ ID NO: 8), (5): m/z=1268.61; Gly-Tyr-Ser-Leu-Gly-Asn-Trp-Val-Cys-Ala-Ala-Lys (SEQ ID NO: 9) and (6): m/z=1045.54; Gly-Thr-Asp-Val-Gln-Ala-Trp-Ile-Arg (SEQ ID NO: 10) are peaks of trypsin-digested peptides derived from lysozyme.

The peaks surrounded by the ellipse of dotted line are the peaks that are smaller by m/z value of 16 or 32 than the peaks of the above objective digested peptides.

As shown in these Figs., only when 3H4NBA was used as a matrix, peaks of the objective peptide fragments containing nitrated tyrosine residue were clearly detected, while peaks of peptide fragments containing a non-nitrated tyrosine residue and the aforementioned peaks smaller by m/z value of 16 or 32 were hardly detected. In other words, by using 3H4NBA as a matrix, only the objective peaks were selectively detected.

### [Example B-5]

In Example 5, using a mixture of peptide whose cysteine is modified with NBS reagent (2-nitrobenzenesulfenyl chloride (MW=189.62): SHIMADZU) and unmodified peptide as a sample to be measured, and using the matrix 3H4NBA of the present invention and the comparative conventional matrix 4-CHCA, measurement was conducted using a mass spectrometer.

The preparing method of the sample to be measured will be described below.

First, 10 µg of peptide IRRP1(Cys-Leu-Lys-Asp-Arg-His-Asp (SEQ ID NO: 11) purchased from BACHEM) was dispensed using 50% acetonitrile aqueous solution containing 0.1% TFA, and lyophilized. This was then dissolved in 15 µl of Milli-Q water (Millipore). The resultant solution was mixed with 35 µl of NBS reagent solution (0.17 mg of NBS reagent was dissolved in 35 µl of acetic acid) and allowed to react for an hour at room temperature. The reaction product was desalted using ZipTip µ-C18 to give a modified peptide sample.

Separately, 10 µg of peptide IRRP1 was dissolved in 15 µl of Milli-Q water. The resultant solution was mixed with 35 µl of acetic acid, left for an hour at room temperature, and desalted with ZipTip µ-C18 to give an unmodified peptide sample.

Equivalent amounts of the modified peptide sample and the unmodified peptide sample were mixed, to prepare a sample to be measured.

The sample to be measured was subjected to measurement by a mass spectrometer while as a matrix, the same matrices as used in Example B-4 were prepared and used. The obtained spectra are shown in Fig. 9. In Fig. 9, the range corresponding to m/z value of 845 to 940 indicated by "x5" is enlarged five times in the vertical direction. In Fig. 9, (a) is a spectrum measured by using 4-CHCA as a matrix, (b) is a spectrum measured by using 3H4NBA as a matrix. Furthermore, the peak indicated by the filled arrow (m/z=1038.40) is a peak of an objective modified peptide sample, the peak indicated by the open arrow (m/z=886.42) is a peak of unmodified peptide sample, and the peak surround by the ellipse of dotted line is a peak that is smaller by m/z value of 16 than the objective peptide peak.

As shown in these Figs., only when 3H4NBA was used as a matrix, peaks of the objective modified peptide sample were clearly detected, while peaks of unmodified peptides and the aforementioned peaks smaller by m/z value of 16 that had been detected using 4-CHCA were not detected. In other words, by using 3H4NBA as a matrix, only the objective peaks were selectively detected.

### [Example B-6]

In this Example, using a peptide containing p-nitro phenylalanine as a sample to be measured, and using the matrix 3H4NBA of the present invention and the comparative conventional matrix 4-CHCA, measurement was conducted using a mass spectrometer.

The preparing method of the sample to be measured will be described below.

10 µg of peptide HIV subIII(His-Lys-Ala-Arg-Val-Leu-Phe^{*}-Glu-Ala-nLeu-Ser-NH₂ (SEQ ID NO: 12); Phe*=p-nitrophenylalanine, nLeu=norleucine, Ser-NH₂=serine whose carboxyl group is amidated; purchased from BACHEM) was dispensed using 50% acetonitrile aqueous solution containing 0.1% TFA and lyophilized. The lyophilized sample was dissolved in 50 µl of 0.1% TFA aqueous solution, and treated with ZipTip µ-C18 to give a sample to be measured.

The sample to be measured was measured by a mass spectrometer while as a matrix, the same matrix as used in Example B-4 were prepared and used. The obtained spectra are shown in Fig. 10. In Fig. 10, (a) is a spectrum measured using 4-CHCA as a matrix, (b) is a spectrum measured using 3H4NBA as a matrix. Furthermore, the peak indicated by the filled arrow (m/z=1314.73) is a peak of an objective peptide HIV subIII, and the peak surround by the ellipse of dotted line is a peak that is smaller by m/z value of 16 than the objective peptide peak.

As shown in these Figs., only when 3H4NBA was used as a matrix, only the objective peak of HIV subIII was detected, while peaks smaller by m/z value of 16 that had been detected using 4-CHCA were not detected. In other words, by using 3H4NBA as a matrix, objective peaks were selectively detected.

### [Example B-7]

In this Example, measurement by a mass spectrometer was conducted using a mixture of ACTH modified with NBS reagent (2-nitrobenzenesulfenyl chloride (MW=189.62): SHIMADZU) and unmodified ACTH as a sample to be measured, and using the matrices of the present invention, 2,4DNA (2,4-dinitroaniline), 2B4,6DNA (2-bromo-4,6-dinitroaniline), 4NA (4-nitroaniline), 4NBA (4-nitrobenzoic acid), 2NP (2-nitrophenol), and 2,5DNP (2,5-dinitrophenol), and the comparative conventional matrix 4-CHCA.

In the following, a preparation method of a sample to be measured will be described. As a sample to be measured, two samples having different concentrations were prepared.

A modified peptide sample (modified ACTH) was prepared in the same manner as in Example B-1 except that purification by chromatography using a C18 column (YMC-Pack Pro C18: YMC) was conducted in place of the desalting treatment by ZipTip µ-C18. Equivalent moles of this modified peptide sample and unmodified peptide sample (unmodified ACTH) were mixed, and dissolved in 50% acetonitrile aqueous solution containing 0.1% TFA so that the respective concentration was 0.5 pmol/µl or 5 pmol/µl, to prepare a sample to be measured.

As the matrix, 2,4DNA, 2B4, 6DNA, 4NA, 4NBA, 2NP, and 2, 5DNP (4NA was purchased from SIGMA and other compounds were purchased from ALDRICH) were each dissolved in 50% acetonitrile aqueous solution containing 0.1% TFA. 4NA, 2NP, and 2,5DNP were used as solutions of 10 mg/ml, and 2,4DNA, 2B4,6DNA, and 4NBA were used as saturated solutions. The comparative conventional 4-CHCA was used as a solution of 10 mg/ml.

Using the above matrices and the above samples to be measured, measurement was conducted by a mass spectrometer. The obtained spectra are shown in Fig. 11 and Fig. 12.

In Fig. 11, (a) is a spectrum obtained by using 4-CHCA as a matrix, (b) is a spectrum obtained by using 2,4DNA as a matrix, and (c) is a spectrum obtained by using 2B4,6DNA as a matrix. These spectra were of the samples to be measured in which the concentrations of the modified peptide and unmodified peptide were respectively adjusted to 0.5 pmol/µl.

In Fig.12, (a) is a spectrum obtained by using 4-CHCA as a matrix, (b) is a spectrum obtained by using 4NA as a matrix, (c) is a spectrum obtained by using 4NBA as a matrix, (d) is a spectrum obtained by using 2NP as a matrix, and (e) is a spectrum obtained by using 2,5DNP as a matrix. These spectra were of the samples to be measured in which the concentrations of the modified peptide and unmodified peptide were respectively adjusted to 5 pmol/µl.

Furthermore, the peak indicated by the filled arrow (m/z=983.37) is a peak of an objective modified peptide sample, the peak indicated by the open arrow (m/z=831.39) is a peak of unmodified peptide sample, and the peaks surround by the ellipse of dotted line are peaks that are smaller by m/z value of 16 or 32 than the objective peptide peak.

As shown in these Figs., in all cases using the matrices of the present invention, peaks of unmodified peptides and the aforementioned peaks smaller by m/z value of 16 or 32 that had been detected using 4-CHCA were not detected, while only the peaks of modified peptides were mainly detected. In other words, it is found that, by using the matrices of the present invention, only the objective peaks were selectively detected.

### [Example B-8]

In this Example, measurement by a mass spectrometer was conducted using a mixture of NBS modified peptides and unmodified peptides as a sample to be measured, and a mixed matrix of 4-CHCA and 3H4NBA; a mixed matrix of 4-CHCA and 3H2NBA (3-hydroxy-2-nitrobenzoic acid); a mixed matrix of 4-CHCA and 2,4DNA; a mixed matrix of 4-CHCA and 4NA; a mixed matrix of 4-CHCA and 4NP (4-nitrophenol); and a matrix of 3H4NBA by itself which are the matrices of the present invention and the comparative conventional matrix 4-CHCA.

Two sample mixtures each having a total weight of 100 µg given by each 25 µg of four purified proteins (ovalbumin, glyceraldehyde-3-phosphate dehydrogenase, lysozyme, and α-lactalbumin, all available from SIGMA) was mixed were prepared.

Samples to be measured were prepared in accordance with a protocol for "¹³CNBS Isotope Labeling Kit" (SHIMADZU) except that solubilization for each mixture and resolubilization for NBS-modified sample mixture were conducted using urea having a final concentration of 8M as a denaturing agent. One sample mixture was modified with a NBS Reagent (heavy) (2-nitro [¹³C₆] benzenesulfenyl chloride) and the other sample mixture was modified with a NBS Reagent (light) (2-nitro[¹²C₆] benzenesulfenyl chloride). Mixing of the both of the modified samples, reduction, alkylation, and trypsin digestion were conducted. The sample after digestion was desalted with ZipTip µ-C18, eluted with 50% acetonitrile aqueous solution containing 0.1% TFA, and the resultant eluate was used as a sample to be measured. The eluate was diluted using 50% acetonitrile aqueous solution containing 0.1% TFA to make a 10-fold dilution, and 0.5 µl of the resultant solution was applied on a target plate.

As a matrix, those prepared in the following manner were used. Using 50% acetonitrile aqueous solution containing 0.1% TFA as a solvent, 4-CHCA, 3H4NBA, 3H2NBA, 2,4DNA, 4NA, and 4NP were each dissolved. 4-CHCA, 3H2NBA, 4NA, and 4NP were prepared into 10 mg/ml solutions, and 3H4NBA and 2,4DNA were prepared into saturated solutions. As to a mixed matrix, the solutions prepared in this manner were mixed in a volume ratio of 1:1. As to the matrix 3H4NBA used by itself and the comparative conventional matrix 4-CHCA, the solutions prepared in this manner were directly used.

On a target plate on which a sample to be measured was applied, which was prepared in advance, 0.5 µL of the matrix solution was added and dried, and then measurement using a mass spectrometer was conducted. The obtained spectra are shown in Fig. 13. In Fig. 13, (a) is a spectrum measured by using a mixed matrix of 4-CHCA and 3H4NBA (+3H4NBA); (b) is a spectrum measured by using a mixed matrix of 4-CHCA and 3H2NBA (+3H2NBA); (c) is a spectrum measured by using a mixed matrix of 4-CHCA and 2,4DNA (+2,4DNA); (d) is a spectrum measured by using a mixed matrix of 4-CHCA and 4NA (+4NA); (e) is a spectrum measured by using a mixed matrix of 4-CHCA and 4NP (+4NP); (f) is a spectrum measured by using a comparative conventional matrix 4-CHCA; (g) is a spectrum measured by using a matrix of 3H4NBA used by itself. Further, it is shown that the pairs of peaks indicated by the filled arrows are of the objective NBS-modified peptides. The pair of peaks of the objective peptide has a difference of m/z value of 6 or 12 corresponding to a mass difference between the two reagents used in the modification, the NBS Reagent (heavy) (2-nitro [¹³C₆] benzenesulfenyl chloride) and the NBS Reagent (light) (2-nitro[¹²C₆] benzenesulfenyl chloride) or to a mass difference between their multiples.

As shown in Fig. 13, when the mixed matrices were used as is (a) to (e), the objective peaks were detected more specifically than the case where 4-CHCA alone was used as is (f). Furthermore, in (a) to (e) using the mixed matrices, the detection sensitivity of the objective peak was better than that of the case using 3H4NBA alone as is (g). In conclusion, (a) to (e) using the mixed matrices of the matrices of the present invention are preferable measuring conditions because the detection sensitivity improves to the same degree as the case using the conventionally used 4-CHCA, while maintaining a specific ionization ability to an objective substance that is achieved when a single compound of the matrices of the present invention as is (g) is used.

The above-described Examples B-1 to B-8 show concrete eight modes within the scope of the present invention, however, the present invention can be carried out in various other modes. Therefore, the above-described Examples are merely illustrative in all respects, and must not be construed as being restrictive. Further, the changes that fall within the equivalents of the claims are all within the scope of the present invention.

Experimental Examples C-1 to C-9 exhibited the effects of the present invention using a part or the whole of the protocol of the third invention are shown below.

### <Experimental Example C-1>

As a model protein, each 25 µg of purified four kinds of proteins (ovalbumin (Ova), glycelaldehyde-3-phosphate dehydrogenase (G3P), lysozyme (Lys) and α-lactalbumin (α-lact), each available from SIGMA) were prepared and mixed together to give a total of 100 µg of Control sample (C).

Two kinds of Samples (S) were prepared. One of Samples (S) was prepared as follows. Namely, 100 µg of protein mixture as same as Sample (C) was solubilized with 0.1 w/v% SDS aqueous solution containing 5 mM EDTA according to the conventional NBS protocol followed by heating at 100°C for 3 minutes, modification using an NBS(light) reagent and desalting using an LH-20 column. It is to be noted that "solubilization" referred in the present Experimental Example and following Experimental Examples is a different step from the solubilization generally conducted for preparing a sample of SDS-PAGE.

The other of Samples (S) was prepared in the following manner. 100 µg of protein mixture as same as Sample (C) was subjected to SDS solubilization in the same manner as described above, and modified with an NBS (light) reagent. Another 100 µg of protein mixture as same as Sample (C) was subjected to SDS solubilization in the same manner as described above, and modified with an equal amount of an NBS(heavy) reagent to the above NBS(light) reagent. The resultant NBS(light)-modified sample and NBS(heavy)-modified sample were mixed and desalted with an LH-20 column.

Two kinds of Samples (G) were separately prepared. These samples were prepared in the same manner as in Samples (S) except that solubilization was conducted using 6 M guanidine hydrochloride aqueous solution containing 5 mM EDTA as in the protocol of the present invention.

Two kinds of Samples (U) were separately prepared. These samples were prepared in the same manner as in Samples (S) except that solubilization was conducted using 8 M urea aqueous solution containing 5 mM EDTA as in the protocol of the present invention.

Samples (C), (S), (G), and (U) obtained in the manner as described above were subjected to electrophoresis. As to Samples (C), an amount corresponding to its 10 µg was developed on Lane 1. As to each of Samples (S), (G), and (U), an amount corresponding to its 1/20 was developed (Lanes 2 to 7).

The result of electrophoresis is shown in Fig. 14. In Fig. 14, lane 1 is for Control sample (C); Lane 2 is for Sample (S) containing only NBS(light)-modified protein; Lane 3 is for Sample (G) containing only NBS(light)-modified protein; Lane 4 is for Sample (U) containing only NBS(light)-modified protein; Lane 5 is for Sample (S) in which equal amounts of NBS(light)-modified protein and NBS(heavy)-modified protein are mixed; Lane 6 is for Sample (G) in which equal amounts of NBS(light)-modified protein and NBS(heavy)-modified protein are mixed; and Lane 7 is for Sample (U) in which equal amounts of NBS(light)-modified protein and NBS(heavy)-modified protein are mixed. As shown by the results of Lanes 3, 4, 6, and 7 in Fig. 14, it is proven to be possible to keep the solubility with least loss of the sample when guanidine hydrochloride or urea serving, both of which are denaturing agents, is used for solubilization.

### <Experimental Example C-2>

100 µg of mouse (C57BL) serum was prepared as Control sample (C).

Two types of Samples (S) were separately prepared. One of Samples (S) was prepared in the following manner. Namely, 100 µg of mouse serum as same as Sample (C) was solubilized with a 0.1 w/v% SDS aqueous solution containing 0.5 mM EDTA followed by heating at 100°C for 3 minutes according to the conventional NBS protocol, and modified with an NBS (light) reagent. Another 100 µg of mouse serum as same as Sample (C) was subjected to SDS solubilization in the same manner as described above, and modified with an equal amount of an NBS(heavy) reagent to the above NBS (light) reagent. The resultant NBS (light)-modified sample and NBS(heavy)-modified sample were mixed together to obtain a modified mixture.

The other of Samples (S) was prepared in the following manner. Namely, from 100 µg of mouse serum, a modified mixture was prepared in the same manner as described above, which was further subjected to desalting by an LH-20 column.

Two types of Samples (G) were separately prepared. These samples were prepared in the same manner as in Samples (S) except that solubilization was conducted using 6 M guanidine hydrochloride aqueous solution containing 5 mM EDTA as in the protocol of the present invention.

Two types of Samples (U) were separately prepared. These samples were prepared in the same manner as in Samples (S) except that solubilization was conducted using 8 M urea aqueous solution containing 5 mM EDTA as in the protocol of the present invention.

Samples (C), (S) (U), and (G) obtained were subjected to electrophoresis. As to Samples (C), an amount corresponding to its 10 µg was developed on Lane 2, and an amount corresponding to its 2 µg was developed on Lane 3. As to each Samples (S), (U), and (G), an amount corresponding to its 1/20 was developed (Lanes 4 to 9).

The result of electrophoresis is shown in Fig. 15. In Fig. 15, Lane 1 is for a molecular weight marker; Lane 2 and 3 are for Control sample (C); Lane 4 is for Sample (S) after modification; Lane 5 is for Sample (S) after modification and desalting; Lane 6 is for Sample (U) after modification; Lane 7 is for Sample (U) after modification and desalting; Lane 8 is for Sample (G) after modification; and Lane 9 is for Sample (G) after modification and desalting. As shown by the results of Lanes 6, 7, 8, and 9 in Fig. 15, it is proven to be possible to keep the solubility with least loss of the sample when guanidine hydrochloride or urea serving, both of which are denaturing agents, is used for solubilization.

### <Experimental Example C-3>

An extract of mouse liver was used as an analyzing sample. With respect to this analyzing sample, solubilization by SDS, modification, desalting, resolubilization by SDS, reduction and alkylation, and digestion were conducted according to the conventional NBS protocol. With respect to another analyzing sample, solubilization by urea, modification, desalting, resolubilization by urea, reduction and alkylation, and digestion were conducted according to the protocol of the present invention. In any modification operation, on the one hand the solubilized sample was modified with an NBS(light) reagent; on the other hand the same amount of solubilized sample was modified with an equal amount of an NBS(heavy) reagent to the NBS(light) reagent; and then the both of the resulting modified samples were mixed. In the following Experimental Examples, modification operation is executed in the same manner. Each obtained sample was subjected to separation of NBS modified peptide using the phenyl column (column available from Amersham Biosciences: HiTrap phenyl). Elution was conducted by a stepwise concentration gradient (concretely, 7 levels of concentrations of 5% interval between 10% to 40%) of acetonitrile. Two fractions were assigned to each concentration.

Each elution fraction (EL1 to EL14) was analyzed using an AXIMA-CFR. The numbers of observed pairs of peaks are listed in Table 1. In the Table, (a) is a result by the method using SDS, and (b) is a result by the method using urea.

**Table 1**

| | (a) | (b) |
|---|---|---|
| | SDS | Urea |
| EL1 | 2 | 8 |
| EL2 | 2 | 11 |
| EL3 | 2 | 17 |
| EL4 | 3 | 20 |
| EL5 | 2 | 25 |
| EL6 | 3 | 16 |
| EL7 | 4 | 18 |
| EL8 | 5 | 24 |
| EL9 | 4 | 12 |
| EL10 | 4 | 10 |
| EL11 | 2 | 5 |
| EL12 | 1 | 1 |
| EL13 | 0 | 3 |
| EL14 | 2 | 4 |
| total | 14 | 76 |

As shown in this Table, a total of 76 pairs of peaks were observed in the method using urea according to the protocol of the present invention, as contrast with a total of 14 pairs of peaks observed in the method using SDS according to the conventional NBS protocol. Further, mass spectra for the fraction EL5 (namely acetonitrile concentration: 20%) in the Table are shown in Fig. 16(a) and (b). The peaks indicated by the arrows in Figures are pairs of peaks of modified peptides.

### <Experimental Example C-4>

A mixture of three kinds of purified proteins (G3P, Lys, and α-lact) was used as a model protein. The mixture was subjected to solubilization by SDS, modification, desalting, resolubilization by SDS, reduction and alkylation, and digestion according to the conventional NBS protocol. Separately the mixture was subjected to solubilization by guanidine hydrochloride, modification, desalting, resolubilization by guanidine hydrochloride, reduction and alkylation, and digestion were conducted according to the protocol of the present invention. Further separately the mixture was subjected to the same operation as described above according to the protocol of the present invention except that urea was used for solubilization. Each obtained sample was subjected to separation of NBS-modified peptide using a phenyl column, and MS analysis was performed.

With regard to the obtained MS spectra, an area ratio of monoisotopic peaks of each pairs of peaks was quantified and compared. Concretely, in each pair of peaks, a relative area of a smaller peak when an area of larger peak was defined as 100 was determined, which was used as an index of quantification to compare both of the obtained spectra. The result is shown in Table 2. As shown in Table 2, when guanidine hydrochloride (GdnHCl) and urea were used according to the protocol of the present invention, an average value of a relative area of peak was respectively 90.0 and 92.0, as compared with an average value of 80.3 obtained by the method using SDS according to the conventional NBS protocol. This shows that quantitativeness is significantly improved by the present invention.

**Table 2**

| | m/z | area ratio of pairs of peaks | | |
|---|---|---|---|---|
| | | GdnHCl | Urea | SDS |
| G3P | 627 | 92.5 | 93.1 | 96.5 |
| | 1916 | 83.7 | 93.1 | 61.8 |
| α-lact | 759 | 91.5 | 93.8 | 98.2 |
| | 1244 | 81.5 | 96.4 | 92.5 |
| | 1353 | 97 | 96.1 | 90.7 |
| Lys | 1198 | 97.3 | 98.9 | 72.3 |
| | 1299 | 93.8 | 80.2 | 78.7 |
| | 1478 | | 95.6 | 63.0 |
| | 1981 | 82.3 | 86 | 68.9 |
| | | | | |
| average | | 90.0 | 92.6 | 80.3 |
| variance | | 37.1 | 30.5 | 186.4 |
| standard deviation | | 6.1 | 6.1 | 13.7 |

Additionally, variance and standard deviation of area ratio of pairs of peaks were determined. The results are also shown in Table 2. As shown in Table 2, variance was respectively 37.1 and 30.5 by the method using guanidine hydrochloride and urea according to the protocol of the present invention, as compared with 186.4 obtained by the method using SDS according to the conventional protocol. On the other hand, standard deviation was respectively 6.1 and 5.5, by the method using guanidine hydrochloride and urea according to the protocol of the present invention, as compared with 13.7 obtained by the method using-SDS according to the conventional protocol. The smaller the values of variance and standard deviation are, the smaller the data variation is. Therefore, it was also demonstrated that the present invention greatly improved data variation.

### <Experimental Example C-5>

Using a mixture of four kinds of purified proteins (Ova, G3P, Lys, and α-lact) as a protein model, solubilization, modification, desalting, resolubilization, reduction and alkylation, and digestion were conducted according to the conventional NBS protocol. On the other hand, using the same protein model, solubilization by urea, modification, desalting, resolubilization by urea, reduction and alkylation, and digestion were conducted according to the protocol of the present invention. Further, both of the resultant samples were fractionated respectively using a phenyl column. From the sample prepared by operating according to the conventional NBS protocol, one fraction was collected, and from the sample prepared by operating according to the protocol of the present invention, a fraction corresponding to the above one fraction was collected. Each collected fraction was analyzed using an AXIMA-CFR. The results are shown in Fig. 17(a) and (b). In Fig. 17, (a) shows a result for the sample obtained according to the conventional NBS protocol, and (b) shows a result for the sample obtained according to the protocol of the present invention. In these Figs., the horizontal axis represents mass-to-charge ratio, and the vertical axis represents a relative intensity of ion. As shown by the results of Fig. 17, a +57 (*m*/*z*) peak indicative of occurrence of alkylation which is a side reaction is detected in the conventional method, while such a peak is not detected in the method of the present invention.

### <Experimental Example C-6>

Using a mixture of four kinds of purified proteins (Ova, G3P, Lys, and α-lact) as a protein model, solubilization, modification, desalting, resolubilization, reduction and alkylation, digestion, and separation of NBS-modified peptides using an enrichment column (LH-20) were conducted according to the conventional NBS protocol. A mass spectra of a representative eluted fraction is shown in Fig. 18. On the other hand, using the same protein model, solubilization by urea, modification, desalting, resolubilization by urea, reduction and alkylation, digestion, and separation of NBS-modified proteins using a phenyl column (column available from Amersham Biosciences: HiTrap phenyl) were conducted according to the protocol of the present invention. A mass spectra of a representative eluted fraction is shown in Fig. 19.

In these Figs., the horizontal axis represents mass-to-charge ratio, and the vertical axis represents a relative intensity of ion. Fig. 18 shows results for the first, third, fifth, seventh, and ninth fractions (Fr.1, Fr.3, Fr.5, Fr.7, and Fr.9) among the total of 10 fractions obtained by the LH-20. Fig. 19 shows results for the first, fourth, seventh, tenth, twelfth, and fourteenth fractions (Fr.1, Fr.4, Fr.7, Fr.10, Fr.12, and Fr.14) among the total of 18 fractions obtained by the phenyl column. As shown by the results of Fig. 18 and Fig. 19, unmodified peptides that are observed a lot around m/z value of 1200 to 1700 in the conventional NBS method were hardly observed in the method of the present invention. Additionally, in Fig. 18, the first, second, fourth, ninth, tenth, twelfth, and thirteenth peptides (indicated by arrows in Fig.) were eluted in almost all of the eluted fractions for measurement, while in Fig. 19, such peptides were eluted while being separated to some extent.

### <Experimental Example C-7>

Using a mixture of four kinds of purified proteins (Ova, G3P, Lys, and α-lact) as a model protein, solubilization by urea, modification, desalting, resolubilization by urea, reduction and alkylation, digestion, and separation of NBS-modified peptides using a phenyl column were conducted according to the protocol of the present invention. Then one fraction eluted from the phenyl column was prepared as a sample for mass spectrometry, and mass spectrometry using an AXIMA-QIT was conducted using respective following three matrices. As matrix, three kinds of matrices: DHB that is conventionally used, and 3-CHCA and 3H4NBA that are used in the present invention were used; and, each of DHB and 3-CHCA was used as a solution of 10 mg/ml and 3H4NBA was used as a saturated solution, respectively in a solvent of 50% acetonitrile aqueous solution containing 0.1% TFA. Equal amounts of the prepared sample and the matrix solution were mixed and subjected to measurement using an AXIMA-QIT. The results are shown in Figs. 20(a) to (c).

In these Figs., the horizontal axis represents mass-to-charge ratio, and the vertical axis represents a relative intensity of ion. The peaks indicated by the arrows (i) to (iii) in Figs. 20(b) and 20(c) are pairs of peaks of modified peptides. As shown in Figs. 20(a) to 20(c), when DHB was used as a matrix (a), NBS-modified peptides were hardly ionized so that they were not detected on the mass spectrum. Contrarily, when 3-CHCA (b) and 3H4NBA (c) were used, NBS-modified proteins were efficiently ionized so that there were detectable on the mass spectra. Further, the result of MS/MS analysis, of the ion for the peak at m/z value of 1198.53 corresponding to an NBS(light)-modified peptide in the pairs of peaks indicated by the arrow (i) obtained in Fig. 20(b), is shown in Fig. 21.

### <Experimental Example C-8>

In the present Experimental Example, measurement was conducted by means of a mass spectrometers using a mixture of peptides modified with NBS reagent and unmodified peptides as a sample to be measured, and using a mixed matrix containing 3H4NBA and 4-CHCA.

The sample to be measured was prepared in the following manner.

Two sample mixtures each having a total weight of 100 µg given by each 25 µg of four purified proteins (ovalbumin, glyceraldehyde-3-phosphate dehydrogenase, lysozyme, and α-lactalbumin, all available from SIGMA) was mixed were prepared. The protocol for "¹³CNBS Isotope Labeling Kit" (SHIMADZU) was followed except that solubilization of each mixture and resolubilization of NBS-modified sample mixture was conducted using urea having a final concentration of 8M as a denaturing agent. Specifically, One sample mixture was labeled-modified with a NBS Reagent (heavy) (2-nitro[¹³C₆] benzenesulfenyl chloride), and the other sample mixture was nonlabeled-modified with a NBS Reagent (light) (2-nitro[¹²C₆] benzenesulfenyl chloride). Mixing of the both of the modified samples, desalting, resolubilization by urea, reduction, alkylation, and trypsin digestion were conducted. The samples after digestion were conducted desalting treatment with ZipTip µ-C18, and eluting with 4 µl of 50% acetonitrile aqueous solution containing 0.1% TFA, to obtain a sample to be measured. 0.5 µl from this sample was applied on a target plate.

The matrix for use was prepared in the following manner. Each of 3H4NBA and 4-CHCA was dissolved in a solvent of 50% acetonitrile aqueous solution containing 0.1% TFA. 3H4NBA was prepared into a saturation solution, and 4-CHCA was prepared into a solution of 10 mg/ml. These solutions thus prepared were mixed with each other in a volume ratio of 1:1 to obtain a mixed matrix solution. On a prepared target plate on which a sample to be measured was applied, 0.5 µL of the mixed matrix solution was added. After drying, measurement was conducted using a MALDI-IT-TOF mass spectrometer having an ion trap (AXIMA-QIT, SHIMADZU) and a MALDI-TOF mass spectrometer without an ion trap (AXIMA-CFR plus, SHIMADZU).

The MS spectra obtained in these measurements are shown in Fig. 22. In Fig. 22, the horizontal axis represents mass-to-charge ratio (m/z), and the vertical axis represents relative intensity of ion (%int.). (a) is a spectrum obtained by using the AXIMA-QIT having an ion trap, and (b) is a spectrum obtained by using the AXIMA-CFR plus without an ion trap. In Fig. 22, the peaks of pairs marked with the arrows come from NBS-modified peptides. Each pair of peaks has a difference of m/z value of 6 that is corresponding to a difference in mass between the two modification reagents, that is, between the NBS Reagent (heavy) (2-nitro [¹³C₆] benzenesulfenyl chloride) and the NBS Reagent (light) (2-nitro[¹²C₆] benzenesulfenyl chloride).

As can be seen by comparison of the spectra of Fig. 22 (a) and (b), almost the same spectrum was obtained. The fact that almost the same spectrum was obtained by a mass spectrometer having an ion trap and by a mass spectrometer without an ion trap indicates that self-disintegration of the analyte is suppressed in measurement using a mass spectrometer having an ion trap. This leads the conclusion that the matrix mixture of the present invention suppresses self-disintegration of an analyte to be measured that occurs during conventional measurement using a mass spectrometer with an ion trap (namely, mass spectrometer requiring relatively long time from ionization to detection of ion) using 4-CHCA alone as a matrix. Further, almost of the detected peaks were pairs of peaks of NBS-modified peptide, which shows that the ability to detect with specificity possessed by the matrix 3H4NBA alone is maintained even in using the mixed matrix.

### <Experimental Example C-9>

In the present Experimental Example, using the same sample to be measured as Experimental Example C-8, a mass spectrometry was conducted with a matrix of 3H4NBA by itself and a mixed matrix of 3H4NBA and 4-CHCA.

Using the same sample to be measured as Experimental Example C-8, 4 µl of eluate was obtained as a sample to be measured in the same manner as Experimental Example 8. The sample to be measured was diluted in 0.1% TFA aqueous solution to make a 1000-fold dilution, and 0.5 µl from the diluted solution was applied on a target plate.

As the matrix, a matrix of 3H4NBA by itself and a mixed matrix in which 3H4NBA and 4-CHCA is combined were used.

The matrix of 3H4NBA by itself was prepared as a saturated solution by dissolving 3H4NBA in 50% acetonitrile aqueous solution containing 0.1% TFA. On a prepared target plate on which a sample to be measured was applied, the 3H4NBA solution was applied. After drying, measurement was conducted using a MALDI-TOF (AXIMA-CFR plus, SHIMADZU).

The mixed matrix of 3H4NBA and 4-CHCA was prepared in the same manner as in Experimental Example C-8. On a prepared target plate on which a sample to be measured was applied, this mixed solution was applied. After drying, measurement was conducted using a MALDI-TOF (AXIMA-CFR plus, SHIMADZU).

The MS spectra obtained in these measurements are shown in Fig. 23. In Fig. 23, the horizontal axis represents mass-to-charge ratio (m/z), and the vertical axis represents relative intensity of ion (%). (a) is a spectrum obtained by using the mixed matrix of 3H4NBA and 4-CHCA, and (b) is a spectrum obtained by using the 3H4NBA matrix. Further, Fig. 23 shows that the pairs of peaks marked with the arrows come from NBS-modified peptides. Each pair of peaks has a difference of m/z value of 6 that is corresponding to a difference in mass between the two modification reagents, that is, between the NBS Reagent (heavy) (2-nitro[¹³C₆] benzenesulfenyl chloride) and a NBS Reagent (light) (2-nitro[¹²C₆] benzenesulfenyl chloride).

As is apparent from comparison of spectra Fig. 23(a) and (b), the pairs of peaks of NBS-modified peptides are detected more sensitively in (a) than (b). This indicates that sensitivity is improved when 3H4NBA is mixed with 4-CHCA as a matrix. It was confirmed that advantages of 4-CHCA that "measurement with high sensitivity can realize" in the condition that "optimum spot on which a laser beam is to be focused can be readily found" are added while keeping the advantage of 3H4NBA that "NBS-modified peptides can be detected selectively by mass spectrometry."

From the combined results of the above Experimental Example C-8 and Experimental Example C-9, it was confirmed that the advantage of 3H4NBA that "NBS-modified peptides can be detected selectively by mass spectrometry" and that "self-disintegration of analyte can be suppressed even if measurement is conducted using an ion trap type MALDI mass spectrometer in which the time from ionization to detection of ion is relatively long" and the advantage of 4-CHCA that "measurement with high sensitivity can realize" in the condition that "optimum spot on which a laser beam is to be focused can be readily found" were achieved simultaneously.

Experimental Examples C-3, C-4, C-5 and C-7 among the above described Experimental Examples show concrete modes within the scope of the present invention, however, the present invention can be carried out in various other modes. Therefore, the above-described Experimental Examples are merely illustrative in all respects, and must not be construed as being restrictive. Further, the changes that fall within the equivalents of the claims are all within the scope of the present invention.

## Claims

1. A method of measuring a peptide with a mass spectrometer having a MALDI (Matrix Assisted Laser Desorption/Ionization) ion source, using α-cyano-3-hydroxycinnamic acid or 3-hydroxy-4-nitrobenzoic acid as a matrix.

2. The method of measuring a peptide according to claim 1, wherein when 3-hydroxy-4-nitrobenzoic acid is used as the matrix, the measuring is performed by using a mixed matrix of 3-hydroxy-4-nitrobenzoic acid and α-cyano-4-hydroxycinnamic acid.

3. The method of measuring a peptide according to claim 1, wherein the peptide is a peptide that is chemically modified with a hydrophobic compound.

4. The method of measuring a peptide according to claim 1, wherein the peptide is a peptide that has an amino acid residue modified with a sulfenyl compound.

5. The method of measuring a peptide according to claim 1, wherein the peptide is a peptide that is derivatized with 2-nitrobenzenesulfenyl chloride.

6. The method of measuring a peptide according to claim 1, performed by a MALDI-IT (Matrix Assisted Laser Desorption/Ionization - Ion Trap) mass spectrometer.

7. The method of measuring a peptide according to claim 1, performed by a MALDI-IT-TOF (Matrix Assisted Laser Desorption/Ionization - Ion Trap - Time of Flight) mass spectrometer.

8. The method of measuring a peptide according to claim 1, performed by a MALDI-FTICR (Matrix Assisted Laser Desorption/Ionization - Fourier Transform Ion Cyclotron Resonance) mass spectrometer.

9. The method of measuring a peptide according to claim 1, wherein the matrix is used as a solution having a concentration of 1 mg/ml to a saturated concentration.

10. The method of measuring a peptide according to claim 2, wherein the α-cyano-4-hydroxycinnamic acid is used as a solution having a concentration of 1 mg/ml to a saturated concentration.

11. The method of measuring a peptide according to claim 10, wherein the solution of 3-hydroxy-4-nitrobenzoic acid and the solution of α-cyano-4-hydroxycinnamic acid are used in combination in a volume ratio of 1:10 to 10:1.

12. A mass spectrometric method comprising:
in a mass spectrometry specifically ionizing a specific substance to be measured contained in a mixture sample containing the specific substance and a substance other than the specific substance by using a matrix that is more likely to ionize the specific substance than the substance other than the specific substance, to selectively measure the specific substance from the mixture.

13. The mass spectrometric method according to claim 12, wherein the specific substance is a substance related to a living organism.

14. The mass spectrometric method according to claim 13, wherein the substance related to a living organism is selected from protein, peptide, sugar, and lipid.

15. The mass spectrometric method according to claim 12, wherein the specific substance is labeled with an isotope.

16. The mass spectrometric method according to claim 12, wherein the matrix can interact with the specific substance via van der Waals interaction.

17. The mass spectrometric method according to claim 12, wherein the specific substance is a π electron containing substance, and the matrix is a π electron containing substance.

18. The mass spectrometric method according to claim 12, wherein the specific substance is a hydrophilic substance, and the matrix is a hydrophilic substance.

19. The mass spectrometric method according to claim 12, wherein the specific substance is a hydrophobic substance, and the matrix is a hydrophobic substance.

20. The mass spectrometric method according to claim 12, wherein the specific substance is a hydrophobic peptide or a hydrophobic protein.

21. The mass spectrometric method according to claim 20, wherein the hydrophobic peptide or the hydrophobic protein has a benzene ring and/or an aromatic ring other than a benzene ring.

22. The mass spectrometric method according to claim 20, wherein the hydrophobic peptide or the hydrophobic protein further has a nitro group.

23. The mass spectrometric method according to claim 20, wherein the hydrophobic peptide or the hydrophobic protein has a nitrobenzenesulfenyl group or a nitrophenyl group.

24. The mass spectrometric method according to claim 20, wherein the hydrophobic peptide or the hydrophobic protein is obtained by chemically modifying a peptide or a protein corresponding to the hydrophobic peptide or the hydrophobic protein by using a hydrophobic compound having a benzene ring, an aromatic ring other than a benzene ring, and/or a nitro group.

25. The mass spectrometric method according to claim 24, wherein the hydrophobic compound is a sulfenyl compound.

26. The mass spectrometric method according to claim 25, wherein the sulfenyl compound is 2-nitrobenzenesulfenyl chloride.

27. The mass spectrometric method according to claim 19, wherein the matrix is a substituted compound of a benzene ring or an aromatic ring other than a benzene ring, having a functional group for transferring electric charges from/to the specific substance to be measured and a functional group for affording hydrophobicity to the matrix molecule itself.

28. The mass spectrometric method according to claim 27, wherein the functional group for transferring electric charges is selected from carboxyl group, hydroxyl group, amino group, sulfate group, nitrate group, and aldehyde group.

29. The mass spectrometric method according to claim 27, wherein the functional group for affording hydrophobicity is nitro group.

30. The mass spectrometric method according to claim 19, wherein the matrix is a nitrobenzoic acid derivative or a nitrophenol derivative.

31. The mass spectrometric method according to claim 19, wherein the matrix is a hydroxynitrobenzoic acid derivative.

32. The mass spectrometric method according to claim 19, wherein the matrix is selected from positional isomers of hydroxynitrobenzoic acid.

33. The mass spectrometric method according to claim 19, wherein the matrix is selected from 4-nitroaniline, 2,4-dinitroaniline, 2-bromo-4,6-dinitroaniline, 4-nitrophenol, 2-nitrophenol, 2,5-dinitrophenol, 4-nitrobenzoic acid, 3-hydroxy-4-nitrobenzoic acid, and 3-hydroxy-2-nitrobenzoic acid.

34. The mass spectrometric method according to claim 19, wherein as the matrix, a mixed matrix combined with α-cyano-4-hydroxycinnamic acid is used.

35. The mass spectrometric method according to claim 19, wherein the matrix is used as a solution of 1 mg/ml to a saturated concentration.

36. The mass spectrometric method according to claim 34, wherein the α-cyano-4-hydroxycinnamic acid is used as a solution of 1 mg/ml to a saturated concentration.

37. The mass spectrometric method according to claim 36, wherein the matrix solution and the solution of α-cyano-4-hydroxycinnamic acid are used in a volume ratio of 1:10 to 10:1.

38. A method for global quantitative analysis of protein comprising the steps of:
(i) preparing two states of protein samples, a Protein sample I for analysis and a control Protein sample II;
(ii) solubilizing the Protein sample I in a solution containing urea as a denaturing agent or in a solution containing guanidine hydrochloride as a denaturing agent, to obtain a solubilized Protein sample I, and
separately solubilizing the Protein sample II in a solution containing urea as a denaturing agent or in a solution containing guanidine hydrochloride as a denaturing agent, to obtain a solubilized Protein sample II;
(iii) subjecting the solubilized Protein sample I to modification reaction using either one of 2-nitro[¹³C₆]benzenesulfenyl chloride and 2-nitro[¹²C₆]benzenesulfenyl chloride, to obtain a modified Protein sample I, and
separately subjecting the solubilized Protein sample II to modification reaction using the other one of 2-nitro[¹³C₆]benzenesulfenyl chloride and 2-nitro[¹²C₆]benzenesulfenyl chloride, to obtain a modified Protein sample II;
(iv) mixing and desalting the modified Protein sample I and the modified Protein sample II, to obtain a desalted protein sample mixture;
(v) resolubilizing the desalted protein sample mixture by using urea or guanidine hydrochloride, to obtain a resolubilized protein sample mixture;
(vi) reducing and alkylating the resolubilized protein sample mixture, to obtain a reduced and alkylated protein sample mixture;
(vii) subjecting the reduced and alkylated protein sample mixture to trypsin digestion in the presence of urea or guanidine hydrochloride, to obtain a peptide mixture containing modified peptide fragments and unmodified peptide fragments;
(viii) separating the peptide mixture using a media having a phenyl group, to obtain enriched modified peptide fragments; and
(ix) subjecting the enriched modified peptide fragments to mass spectrometry.

39. The method according to claim 38, wherein in the step (ix), the mass spectrometry is conducted using α-cyano-3-hydroxycinnamic acid or 3-hydroxy-4-nitrobenzoic acid as a matrix.

40. The method according to claim 38, wherein in the step (ix), when 3-hydroxy-4-nitrobenzoic acid is used as the matrix, the mass spectrometry is conducted using a mixed matrix of 3-hydroxy-4-nitrobenzoic acid and α-cyano-4-hydroxycinnamic acid.

41. The method according to claim 39, wherein the matrix is used as a solution having a concentration of 1 mg/ml to a saturated concentration.

42. The method according to claim 40, wherein α-cyano-4-hydroxycinnamic acid is used as a solution having a concentration of 1 mg/ml to a saturated concentration.

43. The method according to claim 42, wherein the solution of 3-hydroxy-4-nitrobenzoic acid and the solution of α-cyano-4-hydroxycinnamic acid are combined in a volume ratio of 1:10 to 10:1 to be used.

44. A kit containing 2-nitro[¹³C₆]benzenesulfenyl chloride, 2-nitro[¹²C₆]benzenesulfenyl chloride, and a media having a phenyl group.

45. A kit for carrying out the method according to claim 38, containing 2-nitro[¹³C₆]benzenesulfenyl chloride, 2-nitro[¹²C₆]benzenesulfenyl chloride, and a media having a phenyl group.

46. The kit according to claim 44, further containing a denaturing agent.

47. The kit according to claim 44, further containing α-cyano-3-hydroxycinnamic acid, 3-hydroxy-4-nitrobenzoic acid, or α-cyano-4-hydroxycinnamic acid as a matrix, or a mixture of 3-hydroxy-4-nitrobenzoic acid and α-cyano-4-hydroxycinnamic acid as a mixed matrix.

48. The kit according to claim 46, wherein the denaturing agent is urea or guanidine hydrochloride.

49. The kit according to claim 44, further containing at least one selected from the group consisting of a desalting column, filling gel for a desalting column, a reduction reagent, an alkylation reagent, trypsin, and a column for filling the media.
